# EUROPEAN PATENT SPECIFICATION

(11) **EP 1 757 682 B1**
(45) Date of publication and mention of the grant of the patent: **05.01.2011**
(21) Application number: 05748955.1
(22) Date of filing: 07.06.2005
(51) Int. Cl.: C12N 5/10, A01K 67/027, C12N 15/01

(54) **ES CELL MUTATION METHOD AND SYSTEM**
ES-ZELLMUTATIONSVERFAHREN UND -SYSTEM
METHODE ET SYSTEME DE MUTATION DE CELLULES SOUCHES EMBRYONNAIRES

(30) Priority: 08.06.2004 JP 2004170587
(43) Date of publication of application: 28.02.2007
(73) Proprietor: OSAKA UNIVERSITY, Suita-shi, Osaka 565-0871 (JP); Carna Biosciences Inc., Kobe-shi, Hyogo 650-0047 (JP)
(72) Inventor: TAKEDA, Junji, Suita-shi, Osaka 5650-824 (JP); YUSA, Kosuke, Suita-shi, Osaka 5650-821 (JP); HORIE, Kyoji, Hirakata-shi, Osaka 5731-121 (JP)
(74) Representative: Bates, Philip Ian
(86) International application number: PCT/JP2005/010436
(87) International publication number: WO 2005/121324

(56) References cited:
- WO-A1-02/13602
- HARAGUCHI MISAKO ET AL: "Apoptotic protease activating factor 1 (Apaf-1)-independent cell death suppression by Bcl-2" JOURNAL OF EXPERIMENTAL MEDICINE, vol. 191, no. 10, 15 May 2000 (2000-05-15), pages 1709-1720, XP002457010 ISSN: 0022-1007
- CHESTER N ET AL: "Stage-specific apoptosis, developmental delay, and embryonic lethality in ice homozygous for a targeted disruption in the murine Bloom's syndrome gene" GENES AND DEVELOPMENT, COLD SPRING HARBOR LABORATORY PRESS, PLAINVIEW, NY, US, vol. 12, 1998, pages 3382-3393, XP002981418 ISSN: 0890-9369
- CARPENTER ANNE E ET AL: "Systematic genome-wide screens of gene function." NATURE REVIEWS GENETICS, vol. 5, no. 1, January 2004 (2004-01), pages 11-22, XP002457044 ISSN: 1471-0056
- YUSA K ET AL: 'Genome-wide phenotype analysis in ES cells by regulated disruption of Bloom's syndrome gene.' NATURE. vol. 429, no. 6994, 24 June 2004, pages 896 - 899, XP002996915
- YUSA K ET AL: 'ES cell library bearing bi-allelic mutations.' IGAKU NO AYUMI vol. 210, no. 13, 25 September 2004, pages 1077 - 1078, XP002996916
- GOSS K H ET AL: 'Enhanced Tumor Formation in Mice Heterozygous for Blm Mutation.' SCIENCE. vol. 297, no. 5589, 20 September 2002, pages 2051 - 2053, XP002996917
- WU L ET AL: 'The Bloom's syndrome helicase suppresses crossing over during homologous recombination.' NATURE. vol. 426, no. 6968, December 2003, pages 870 - 874, XP002996918

### SEQUENCE LISTING

<110> Osaka University
   Carna Biosciences Inc.
   Junji Takeda
   Kosuke Yusa
   Kyoji Horie
<120> Method and System for mutating ES cells
<130> CN001PCT
<150> JP 2004-170587
   <151> 2004-06-08
<160> 5
<170> PatentIn version 3.2
<210> 1
   <211> 4251
   <212> DNA
   <213> Mus musculus
<220>
   <221> CDS
   <222> (1)..(4251)
<400> 1
   atg gct gct gtt cct ctg aac aat cta caa gaa caa cta cag cga cac      48
   Met Ala Ala Val Pro Leu Asn Asn Leu Gln Glu Gln Leu Gln Arg His
   1               5                   10                  15
   tca gcc aga aaa ctt aat aat caa ccc agc ctt tca aaa cca aaa tct      96
   Ser Ala Arg Lys Leu Asn Asn Gln Pro Ser Leu Ser Lys Pro Lys Ser
               20                  25                  30
   tta ggt ttt act ttt aaa aag aaa aca tca gag ggt gat gtg tct gtc     144
   Leu Gly Phe Thr Phe Lys Lys Lys Thr Ser Glu Gly Asp Val Ser Val
           35                  40                  45
   act agt gtg tcc gta gta aaa aca cct gcg tta agt gat aaa gat gtg     192
   Thr Ser Val Ser Val Val Lys Thr Pro Ala Leu Ser Asp Lys Asp Val
       50                  55                  60
   aac gtg tct gag gcc ttt tca ttc act gag tct cca ctc cac aaa cca     240
   Asn Val Ser Glu Ala Phe Ser Phe Thr Glu Ser Pro Leu His Lys Pro
   65                  70                  75                  80
   aag cag cag gca aag att gaa ggc ttc ttt aaa cat ttc cct gga agg     288
   Lys Gln Gln Ala Lys Ile Glu Gly Phe Phe Lys His Phe Pro Gly Arg
                   85                  90                  95
   cag caa agc aag ggg acc tgc tct gag ccg tca ctg ccg gcc acg gta     336
   Gln Gln Ser Lys Gly Thr Cys Ser Glu Pro Ser Leu Pro Ala Thr Val
               100                 105                 110
   cag act gct cag gac act ttg tgc act acc ccc aaa acc ccc act gcg     384
   Gln Thr Ala Gln Asp Thr Leu Cys Thr Thr Pro Lys Thr Pro Thr Ala
           115                 120                 125
   aag aaa ctg ccc gtg gct gtt ttc aag aaa tta gaa ttt agt tct tct       432
   Lys Lys Leu Pro Val Ala Val Phe Lys Lys Leu Glu Phe Ser Ser Ser
       130                 135                 140
   gca gac tcc ctc agt gac tgg gct gat atg gat gac ttt gat atg tca       480
   Ala Asp Ser Leu Ser Asp Trp Ala Asp Met Asp Asp Phe Asp Met Ser
   145                 150                 155                 160
   gca tca gat gcg ttt gct tca ctg gct aaa aat cct gcc aca aga gta       528
   Ala Ser Asp Ala Phe Ala Ser Leu Ala Lys Asn Pro Ala Thr Arg Val
                   165                 170                 175
   agc acc gct cag aaa atg aaa aag act aag aga aac ttc ttt aaa cca       576
   Ser Thr Ala Gln Lys Met Lys Lys Thr Lys Arg Asn Phe Phe Lys Pro
               180                 185                 190
   cca cct cgt aaa gcc aat gca gta aag act gac ttg act cct ccc tcc       624
   Pro Pro Arg Lys Ala Asn Ala Val Lys Thr Asp Leu Thr Pro Pro Ser
           195                 200                 205
   ccc gaa tgc ctg caa gtg gat tta acg aag gaa tcg gag gag gag gag       672
   Pro Glu Cys Leu Gln Val Asp Leu Thr Lys Glu Ser Glu Glu Glu Glu
       210                 215                 220
   gag gag gag gag gag gcg gag ggg gcg gac tgc ctg agc agg gat gtg       720
   Glu Glu Glu Glu Glu Ala Glu Gly Ala Asp Cys Leu Ser Arg Asp Val
   225                 230                 235                 240
   atc tgc att gac aat gat tct gct tct gaa gag ctc acg gag aaa gac       768
   Ile Cys Ile Asp Asn Asp Ser Ala Ser Glu Glu Leu Thr Glu Lys Asp
                   245                 250                 255
   acg cag gaa agc cag tct ttg aaa gct cac ttg gga gct gaa aga ggt       816
   Thr Gln Glu Ser Gln Ser Leu Lys Ala His Leu Gly Ala Glu Arg Gly
               260                 265                 270
   gac agt gaa aag aag agc cat gaa gac gaa gct gtg ttc cat tca gtt       864
   Asp Ser Glu Lys Lys Ser His Glu Asp Glu Ala Val Phe His Ser Val
           275                 280                 285
   cag aac act gaa tac ttt gaa cac aat gac aat gat tat gat ata gat       912
   Gln Asn Thr Glu Tyr Phe Glu His Asn Asp Asn Asp Tyr Asp Ile Asp
       290                 295                 300
   ttt gtt cca cct tct cca gaa gaa atc atc tcc act gct tct tcc tcg       960
   Phe Val Pro Pro Ser Pro Glu Glu Ile Ile Ser Thr Ala Ser Ser Ser
   305                 310                 315                 320
   ttg aaa tgt tcc agt atg tta aag gat ctt gat gac tct gac aaa gaa      1008
   Leu Lys Cys Ser Ser Met Leu Lys Asp Leu Asp Asp Ser Asp Lys Glu
                   325                 330                 335
   aag ggc att ctt agc acc tca gaa gag ctt ctg tca aaa cca gag gaa      1056
   Lys Gly Ile Leu Ser Thr Ser Glu Glu Leu Leu Ser Lys Pro Glu Glu
               340                 345                 350
   atg acc aca cac aag tct gat gca gga acc agt aaa gac tgt gat gcc      1104
   Met Thr Thr His Lys Ser Asp Ala Gly Thr Ser Lys Asp Cys Asp Ala
           355                 360                 365
   cag cag ata cgc ata cag cag cag ctt att cat gtg atg gag cac atc      1152
   Gln Gln Ile Arg Ile Gln Gln Gln Leu Ile His Val Met Glu His Ile
       370                 375                 380
   tgt aag tta gtt gat act gtt cct act gat gaa ctg gaa gct ttg aat      1200
   Cys Lys Leu Val Asp Thr Val Pro Thr Asp Glu Leu Glu Ala Leu Asn
   385                 390                 395                 400
   tgt ggg acc gaa ttg ctt caa caa cga aac ata agg agg aag ctc cta      1248
   Cys Gly Thr Glu Leu Leu Gln Gln Arg Asn Ile Arg Arg Lys Leu Leu
                   405                 410                 415
   gct gaa gca ggt ttt aat gga aat gac gtc aga ctt ctg ggt tct ctg      1296
   Ala Glu Ala Gly Phe Asn Gly Asn Asp Val Arg Leu Leu Gly Ser Leu
               420                 425                 430
   tgg agg cac agg cct gat tca ctt gat aac aca gtg cag ggc gac tcc      1344
   Trp Arg His Arg Pro Asp Ser Leu Asp Asn Thr Val Gln Gly Asp Ser
           435                 440                 445
   tgc cct gtg ggg cat cct aat aaa gag tta aat tct cca tac ctt ctc      1392
   Cys Pro Val Gly His Pro Asn Lys Glu Leu Asn Ser Pro Tyr Leu Leu
       450                 455                 460
   tca cat tcc cct tcc act gag gaa tgt tta ccc acc acc act cca gga      1440
   Ser His Ser Pro Ser Thr Glu Glu Cys Leu Pro Thr Thr Thr Pro Gly
   465                 470                 475                 480
   aag aca gga ttc tca gcc acc ccg aag aat ctc ttt gaa agg ccg tta      1488
   Lys Thr Gly Phe Ser Ala Thr Pro Lys Asn Leu Phe Glu Arg Pro Leu
                   485                 490                 495
   ttg aat tcc cat tta cag aag tcc ttt gta agt agc aac tgg gct gaa      1536
   Leu Asn Ser His Leu Gln Lys Ser Phe Val Ser Ser Asn Trp Ala Glu
               500                 505                 510
   aca cca agg atg gaa aac agg aac gaa agc act gac ttc cca ggg agt      1584
   Thr Pro Arg Met Glu Asn Arg Asn Glu Ser Thr Asp Phe Pro Gly Ser
           515                 520                 525
   gtt ctc acc agc acc act gtg aaa gct cag agt aaa caa gct gct tca      1632
   Val Leu Thr Ser Thr Thr Val Lys Ala Gln Ser Lys Gln Ala Ala Ser
       530                 535                 540
   gga tgg aac gta gag aga cac ggc cag gct tcc tat gat atc gat aac      1680
   Gly Trp Asn Val Glu Arg His Gly Gln Ala Ser Tyr Asp Ile Asp Asn
   545                 550                 555                 560
   ttt aat att gat gac ttt gat gat gat gat gat gat gat gac tgg gaa      1728
   Phe Asn Ile Asp Asp Phe Asp Asp Asp Asp Asp Asp Asp Asp Trp Glu
                   565                 570                 575
   aac ata atg cac aat ttt cca gcc agc aaa tct tcc aca gcc acc tac      1776
   Asn Ile Met His Asn Phe Pro Ala Ser Lys Ser Ser Thr Ala Thr Tyr
               580                 585                 590
   cca ccc atc aag gaa ggt ggg cca gtt aaa tct ctc tca gaa agg att      1824
   Pro Pro Ile Lys Glu Gly Gly Pro Val Lys Ser Leu Ser Glu Arg Ile
           595                 600                 605
   tct tca gcc aag gca aag ttt ctt cca gtg gta tca acc gct caa aat      1872
   Ser Ser Ala Lys Ala Lys Phe Leu Pro Val Val Ser Thr Ala Gln Asn
       610                 615                 620
   aca aac ctc tca gag tca att cag aat tgc tct gat aag ctg gcc caa      1920
   Thr Asn Leu Ser Glu Ser Ile Gln Asn Cys Ser Asp Lys Leu Ala Gln
   625                 630                 635                 640
   aat tta tca tca aaa aat cca aaa cat gaa cat ttt caa agt ctt aat      1968
   Asn Leu Ser Ser Lys Asn Pro Lys His Glu His Phe Gln Ser Leu Asn
                   645                 650                 655
   ttt cct cat aca aaa gaa atg atg aag att ttc cat aag aaa ttt ggc      2016
   Phe Pro His Thr Lys Glu Met Met Lys Ile Phe His Lys Lys Phe Gly
               660                 665                 670
   ttg cat aat ttt aga act aat cag cta gag gcg atc aat gct gcg ctg      2064
   Leu His Asn Phe Arg Thr Asn Gln Leu Glu Ala Ile Asn Ala Ala Leu
           675                 680                 685
   ctt ggt gaa gac tgc ttt atc cta atg ccc act gga gga ggt aaa agt      2112
   Leu Gly Glu Asp Cys Phe Ile Leu Met Pro Thr Gly Gly Gly Lys Ser
       690                 695                 700
   ttg tgc tac cag ctc cct gcc tgt gtt tct cct ggg gtc aca att gtc      2160
   Leu Cys Tyr Gln Leu Pro Ala Cys Val Ser Pro Gly Val Thr Ile Val
   705                 710                 715                 720
   att tct ccc ttg aga tca cta ata gta gat caa gtc caa aag ctg act      2208
   Ile Ser Pro Leu Arg Ser Leu Ile Val Asp Gln Val Gln Lys Leu Thr
                   725                 730                 735
   tcc ttt gat att cca gct aca tat ctg aca ggg gat aag act gac tca      2256
   Ser Phe Asp Ile Pro Ala Thr Tyr Leu Thr Gly Asp Lys Thr Asp Ser
               740                 745                 750
   gaa gct gca aat att tac ctc caa tta tcc aaa aaa gac cca atc atc      2304
   Glu Ala Ala Asn Ile Tyr Leu Gln Leu Ser Lys Lys Asp Pro Ile Ile
           755                 760                 765
   aag ctt ttg tat gtt act cca gag aag gtc tgt gca agt aac agg ctg      2352
   Lys Leu Leu Tyr Val Thr Pro Glu Lys Val Cys Ala Ser Asn Arg Leu
       770                 775                 780
   att tct act ctg gag aat ctg tat gag cgg aag ctc ttg gca cgt ttt      2400
   Ile Ser Thr Leu Glu Asn Leu Tyr Glu Arg Lys Leu Leu Ala Arg Phe
   785                 790                 795                 800
   gtc att gat gaa gcg cat tgt gtg agt cag tgg ggt cat gat ttt cgt      2448
   Val Ile Asp Glu Ala His Cys Val Ser Gln Trp Gly His Asp Phe Arg
                   805                 810                 815
   caa gat tac aaa agg atg aat atg ctt cgc cag aag ttt cct tct gtt      2496
   Gln Asp Tyr Lys Arg Met Asn Met Leu Arg Gln Lys Phe Pro Ser Val
               820                 825                 830
   cca gtg atg gcc ctc acg gcc aca gcg aac ccc agg gtc cag aag gac      2544
   Pro Val Met Ala Leu Thr Ala Thr Ala Asn Pro Arg Val Gln Lys Asp
           835                 840                 845
   atc ctc act cag ctg aag atc ctc aga cct cag gtg ttt agc atg agc      2592
   Ile Leu Thr Gln Leu Lys Ile Leu Arg Pro Gln Val Phe Ser Met Ser
       850                 855                 860
   ttt aac aga cac aat ctg aag tac tat gta tta ccc aag aag ccc aaa      2640
   Phe Asn Arg His Asn Leu Lys Tyr Tyr Val Leu Pro Lys Lys Pro Lys
   865                 870                 875                 880
   aaa gta gca ttt gat tgc cta gag tgg atc aga aag cat cac cct tat      2688
   Lys Val Ala Phe Asp Cys Leu Glu Trp Ile Arg Lys His His Pro Tyr
                   885                 890                 895
   gac tcg ggg ata att tac tgc ctc tcc agg agg gaa tgt gac aca atg      2736
   Asp Ser Gly Ile Ile Tyr Cys Leu Ser Arg Arg Glu Cys Asp Thr Met
               900                 905                 910
   gct gac act tta cag aga gaa ggc ctg gct gcc ctg gct tac cat gcg      2784
   Ala Asp Thr Leu Gln Arg Glu Gly Leu Ala Ala Leu Ala Tyr His Ala
           915                 920                 925
   ggc ctc agt gac tct gcc aga gat gag gtg cag cac aag tgg atc aac      2832
   Gly Leu Ser Asp Ser Ala Arg Asp Glu Val Gln His Lys Trp Ile Asn
       930                 935                 940
   cag gac aac tgc cag gtt atc tgt gcg aca att gcg ttt gga atg gga      2880
   Gln Asp Asn Cys Gln Val Ile Cys Ala Thr Ile Ala Phe Gly Met Gly
   945                 950                 955                 960
   att gac aaa cct gac gtg cga ttt gtg att cat gca tct ctt cct aaa      2928
   Ile Asp Lys Pro Asp Val Arg Phe Val Ile His Ala Ser Leu Pro Lys
                   965                 970                 975
   tct atg gag ggt tat tat caa gaa tcc ggc cga gct gga aga gat ggg      2976
   Ser Met Glu Gly Tyr Tyr Gln Glu Ser Gly Arg Ala Gly Arg Asp Gly
               980                 985                 990
   gaa ata tct cac tgc gtg ctt ttc  tat aca tat cat gat  gtg acc aga    3024
   Glu Ile Ser His Cys Val Leu Phe  Tyr Thr Tyr His Asp  Val Thr Arg
           995                 1000                1005
   ctg aag  aga ctt ata atg atg  gaa aaa gat gga aac  tat cat acg      3069
   Leu Lys  Arg Leu Ile Met Met  Glu Lys Asp Gly Asn  Tyr His Thr
       1010                1015                1020
   aag gaa  act cac gtc aat aat  cta tat agc atg gta  cat tac tgt      3114
   Lys Glu  Thr His Val Asn Asn  Leu Tyr Ser Met Val  His Tyr Cys
       1025                1030                1035
   gaa aac  ata acg gaa tgc aga  aga ata cag ctt tta  gct tac ttt      3159
   Glu Asn  Ile Thr Glu Cys Arg  Arg Ile Gln Leu Leu  Ala Tyr Phe
       1040                1045                1050
   ggt gaa  aaa gga ttc aac cct  gat ttt tgt aag aaa  tac cca gat      3204
   Gly Glu  Lys Gly Phe Asn Pro  Asp Phe Cys Lys Lys  Tyr Pro Asp
       1055                1060                1065
   gtt tct  tgt gac aat tgc tgt  aaa aca aag gat tat  aaa aca aaa      3249
   Val Ser  Cys Asp Asn Cys Cys  Lys Thr Lys Asp Tyr  Lys Thr Lys
       1070                1075                1080
   gat gtg  act gat gac gtg aaa  aat att ata aga ttt  gtt caa gaa      3294
   Asp Val  Thr Asp Asp Val Lys  Asn Ile Ile Arg Phe  Val Gln Glu
       1085                1090                1095
   cac agt  tca tca cca gga aca  aga aat ata gga cct  gct gga aga       3339
   His Ser  Ser Ser Pro Gly Thr  Arg Asn Ile Gly Pro  Ala Gly Arg
       1100                 1105                 1110
   ttt act  ctg aac atg ctg gtc  gac att ttc ttg ggg  agc aag agt       3384
   Phe Thr  Leu Asn Met Leu Val  Asp Ile Phe Leu Gly  Ser Lys Ser
       1115                 1120                 1125
   gca aaa  gtt aag tct gga ata  ttt gga aag ggg act  aca tat tca       3429
   Ala Lys  Val Lys Ser Gly Ile  Phe Gly Lys Gly Thr  Thr Tyr Ser
       1130                 1135                 1140
   cga cat  aat gcc gaa aga ctt  ttt aaa aag ctg att  cta gac aaa       3474
   Arg His  Asn Ala Glu Arg Leu  Phe Lys Lys Leu Ile  Leu Asp Lys
       1145                 1150                 1155
   atc ctg  gat gaa gac tta tat  atc aat gcc aat gac  caa cca att       3519
   Ile Leu  Asp Glu Asp Leu Tyr  Ile Asn Ala Asn Asp  Gln Pro Ile
       1160                 1165                 1170
   gcc tat  gtg atg cta gga aca  aaa gcc cac agt gtg  ctg agt ggc       3564
   Ala Tyr  Val Met Leu Gly Thr  Lys Ala His Ser Val  Leu Ser Gly
       1175                 1180                 1185
   cac ttg  aag gtg gac ttc atg  gaa acg gaa aat tcc  agc agt att       3609
   His Leu  Lys Val Asp Phe Met  Glu Thr Glu Asn Ser  Ser Ser Ile
       1190                 1195                 1200
   aaa aaa  caa aaa gct tta gtg  gcc aaa gta tcc cag  aga gaa gag       3654
   Lys Lys  Gln Lys Ala Leu Val  Ala Lys Val Ser Gln  Arg Glu Glu
       1205                 1210                 1215
   gta gtt  aag aaa tgt ctt gga  gaa ctt aca gag gtc  tgc aaa ttg       3699
   Val Val  Lys Lys Cys Leu Gly  Glu Leu Thr Glu Val  Cys Lys Leu
       1220                 1225                 1230
   ctg ggg  aaa gtc ttt ggt gtc  cat tac ttc aat att  ttt aat aca       3744
   Leu Gly  Lys Val Phe Gly Val  His Tyr Phe Asn Ile  Phe Asn Thr
       1235                 1240                 1245
   gcc aca  ctc aaa aag ctt gca  gaa tct tta tct tct  gat cct gag       3789
   Ala Thr  Leu Lys Lys Leu Ala  Glu Ser Leu Ser Ser  Asp Pro Glu
       1250                 1255                 1260
   gtt ttg  ctt cag att gat ggt  gtt acc gaa gac aag  ctg gaa aaa       3834
   Val Leu  Leu Gln Ile Asp Gly  Val Thr Glu Asp Lys  Leu Glu Lys
       1265                 1270                 1275
   tat ggt  gca gaa gtg att cca  gta tta cag aag tac  tca gaa tgg       3879
   Tyr Gly  Ala Glu Val Ile Pro  Val Leu Gln Lys Tyr  Ser Glu Trp
       1280                 1285                 1290
   aca gtg  cca gct gag gat ggt  tcc cca ggc gcc aga  ggc gcc cca       3924
   Thr Val  Pro Ala Glu Asp Gly  Ser Pro Gly Ala Arg  Gly Ala Pro
       1295                 1300                 1305
   gag gac  act gag gag gag gag  gag gaa gcg cct gta  tct tct cac       3969
   Glu Asp  Thr Glu Glu Glu Glu  Glu Glu Ala Pro Val  Ser Ser His
       1310                 1315                 1320
   tac ttt  gca aat caa act aga  aat gaa aga aag aga  aag aaa atg       4014
   Tyr Phe  Ala Asn Gln Thr Arg  Asn Glu Arg Lys Arg  Lys Lys Met
       1325                 1330                 1335
   tca gcc  acc cat aag ccc aag  agg aga aga act agt  tac ggt ggc       4059
   Ser Ala  Thr His Lys Pro Lys  Arg Arg Arg Thr Ser  Tyr Gly Gly
       1340                 1345                 1350
   ttc aga  gca aag ggg ggc tct  act aca tgc aga aaa  acg act tct       4104
   Phe Arg  Ala Lys Gly Gly Ser  Thr Thr Cys Arg Lys  Thr Thr Ser
       1355                 1360                 1365
   aaa agt  aaa ttc tat ggt gta  act gga tcc cgc tcg  gcc tca tgt       4149
   Lys Ser  Lys Phe Tyr Gly Val  Thr Gly Ser Arg Ser  Ala Ser Cys
       1370                 1375                 1380
   gct tct  cag gca aca tca tca  gcc agt aga aaa ctg  ggg att atg       4194
   Ala Ser  Gln Ala Thr Ser Ser  Ala Ser Arg Lys Leu  Gly Ile Met
       1385                 1390                 1395
   gct cct  cca aag cct gta aat  aga acg ttc ctc agg  cct tca tat       4239
   Ala Pro  Pro Lys Pro Val Asn  Arg Thr Phe Leu Arg  Pro Ser Tyr
       1400                 1405                 1410
   gcc ttc  tcc taa                                                    4251
   Ala Phe  Ser
       1415
<210> 2
   <211> 1416
   <212> PRT
   <213> Mus musculus
<400> 2
   Met Ala Ala Val Pro Leu Asn Asn Leu Gln Glu Gln Leu Gln Arg His
   1               5                   10                  15
   Ser Ala Arg Lys Leu Asn Asn Gln Pro Ser Leu Ser Lys Pro Lys Ser
               20                  25                  30
   Leu Gly Phe Thr Phe Lys Lys Lys Thr Ser Glu Gly Asp Val Ser Val
           35                  40                  45
   Thr Ser Val Ser Val Val Lys Thr Pro Ala Leu Ser Asp Lys Asp Val
       50                  55                  60
   Asn Val Ser Glu Ala Phe Ser Phe Thr Glu Ser Pro Leu His Lys Pro
   65                  70                  75                  80
   Lys Gln Gln Ala Lys Ile Glu Gly Phe Phe Lys His Phe Pro Gly Arg
                   85                  90                  95
   Gln Gln Ser Lys Gly Thr Cys Ser Glu Pro Ser Leu Pro Ala Thr Val
                   100                 105                 110
   Gln Thr Ala Gln Asp Thr Leu Cys Thr Thr Pro Lys Thr Pro Thr Ala
           115                 120                 125
   Lys Lys Leu Pro Val Ala Val Phe Lys Lys Leu Glu Phe Ser Ser Ser
       130                 135                 140
   Ala Asp Ser Leu Ser Asp Trp Ala Asp Met Asp Asp Phe Asp Met Ser
   145                 150                 155                 160
   Ala Ser Asp Ala Phe Ala Ser Leu Ala Lys Asn Pro Ala Thr Arg Val
                   165                 170                 175
   Ser Thr Ala Gln Lys Met Lys Lys Thr Lys Arg Asn Phe Phe Lys Pro
               180                 185                 190
   Pro Pro Arg Lys Ala Asn Ala Val Lys Thr Asp Leu Thr Pro Pro Ser
           195                 200                 205
   Pro Glu Cys Leu Gln Val Asp Leu Thr Lys Glu Ser Glu Glu Glu Glu
       210                 215                 220
   Glu Glu Glu Glu Glu Ala Glu Gly Ala Asp Cys Leu Ser Arg Asp Val
   225                 230                 235                 240
   Ile Cys Ile Asp Asn Asp Ser Ala Ser Glu Glu Leu Thr Glu Lys Asp
                   245                 250                 255
   Thr Gln Glu Ser Gln Ser Leu Lys Ala His Leu Gly Ala Glu Arg Gly
               260                 265                 270
   Asp Ser Glu Lys Lys Ser His Glu Asp Glu Ala Val Phe His Ser Val
           275                 280                 285
   Gln Asn Thr Glu Tyr Phe Glu His Asn Asp Asn Asp Tyr Asp Ile Asp
       290                 295                 300
   Phe Val Pro Pro Ser Pro Glu Glu Ile Ile Ser Thr Ala Ser Ser Ser
   305                 310                 315                 320
   Leu Lys Cys Ser Ser Met Leu Lys Asp Leu Asp Asp Ser Asp Lys Glu
                   325                 330                 335
   Lys Gly Ile Leu Ser Thr Ser Glu Glu Leu Leu Ser Lys Pro Glu Glu
               340                 345                 350
   Met Thr Thr His Lys Ser Asp Ala Gly Thr Ser Lys Asp Cys Asp Ala
   355 360 365
   Gln Gln Ile Arg Ile Gln Gln Gln Leu Ile His Val Met Glu His Ile
   370 375 380
   Cys Lys Leu Val Asp Thr Val Pro Thr Asp Glu Leu Glu Ala Leu Asn
   385 390 395 400
   Cys Gly Thr Glu Leu Leu Gln Gln Arg Asn Ile Arg Arg Lys Leu Leu
   405 410 415
   Ala Glu Ala Gly Phe Asn Gly Asn Asp Val Arg Leu Leu Gly Ser Leu
   420 425 430
   Trp Arg His Arg Pro Asp Ser Leu Asp Asn Thr Val Gln Gly Asp Ser
   435 440 445
   Cys Pro Val Gly His Pro Asn Lys Glu Leu Asn Ser Pro Tyr Leu Leu
   450 455 460
   Ser His Ser Pro Ser Thr Glu Glu Cys Leu Pro Thr Thr Thr Pro Gly
   465 470 475 480
   Lys Thr Gly Phe Ser Ala Thr Pro Lys Asn Leu Phe Glu Arg Pro Leu
   485 490 495
   Leu Asn Ser His Leu Gln Lys Ser Phe Val Ser Ser Asn Trp Ala Glu
   500 505 510
   Thr Pro Arg Met Glu Asn Arg Asn Glu Ser Thr Asp Phe Pro Gly Ser
   515 520 525
   Val Leu Thr Ser Thr Thr Val Lys Ala Gln Ser Lys Gln Ala Ala Ser
   530 535 540
   Gly Trp Asn Val Glu Arg His Gly Gln Ala Ser Tyr Asp Ile Asp Asn
   545 550 555 560
   Phe Asn Ile Asp Asp Phe Asp Asp Asp Asp Asp Asp Asp Asp Trp Glu
   565 570 575
   Asn Ile Met His Asn Phe Pro Ala Ser Lys Ser Ser Thr Ala Thr Tyr
   580 585 590
   Pro Pro Ile Lys Glu Gly Gly Pro Val Lys Ser Leu Ser Glu Arg Ile
           595                 600                 605
   Ser Ser Ala Lys Ala Lys Phe Leu Pro Val Val Ser Thr Ala Gln Asn
       610                 615                 620
   Thr Asn Leu Ser Glu Ser Ile Gln Asn Cys Ser Asp Lys Leu Ala Gln
   625                 630                 635                 640
   Asn Leu Ser Ser Lys Asn Pro Lys His Glu His Phe Gln Ser Leu Asn
                   645                 650                 655
   Phe Pro His Thr Lys Glu Met Met Lys Ile Phe His Lys Lys Phe Gly
               660                 665                 670
   Leu His Asn Phe Arg Thr Asn Gln Leu Glu Ala Ile Asn Ala Ala Leu
           675                 680                 685
   Leu Gly Glu Asp Cys Phe Ile Leu Met Pro Thr Gly Gly Gly Lys Ser
       690                 695                 700
   Leu Cys Tyr Gln Leu Pro Ala Cys Val Ser Pro Gly Val Thr Ile Val
   705                 710                 715                 720
   Ile Ser Pro Leu Arg Ser Leu Ile Val Asp Gln Val Gln Lys Leu Thr
                   725                 730                 735
   Ser Phe Asp Ile Pro Ala Thr Tyr Leu Thr Gly Asp Lys Thr Asp Ser
               740                 745                 750
   Glu Ala Ala Asn Ile Tyr Leu Gln Leu Ser Lys Lys Asp Pro Ile Ile
           755                 760                 765
   Lys Leu Leu Tyr Val Thr Pro Glu Lys Val Cys Ala Ser Asn Arg Leu
       770                 775                 780
   Ile Ser Thr Leu Glu Asn Leu Tyr Glu Arg Lys Leu Leu Ala Arg Phe
   785                 790                 795                 800
   Val Ile Asp Glu Ala His Cys Val Ser Gln Trp Gly His Asp Phe Arg
                   805                 810                 815
   Gln Asp Tyr Lys Arg Met Asn Met Leu Arg Gln Lys Phe Pro Ser Val
               820                 825                 830
   Pro Val Met Ala Leu Thr Ala Thr Ala Asn Pro Arg Val Gln Lys Asp
   835 840 845
   Ile Leu Thr Gln Leu Lys Ile Leu Arg Pro Gln Val Phe Ser Met Ser
   850 855 860
   Phe Asn Arg His Asn Leu Lys Tyr Tyr Val Leu Pro Lys Lys Pro Lys
   865 870 875 880
   Lys Val Ala Phe Asp Cys Leu Glu Trp Ile Arg Lys His His Pro Tyr
   885 890 895
   Asp Ser Gly Ile Ile Tyr Cys Leu Ser Arg Arg Glu Cys Asp Thr Met
   900 905 910
   Ala Asp Thr Leu Gln Arg Glu Gly Leu Ala Ala Leu Ala Tyr His Ala
   915 920 925
   Gly Leu Ser Asp Ser Ala Arg Asp Glu Val Gln His Lys Trp Ile Asn
   930 935 940
   Gln Asp Asn Cys Gln Val Ile Cys Ala Thr Ile Ala Phe Gly Met Gly
   945 950 955 960
   Ile Asp Lys Pro Asp Val Arg Phe Val Ile His Ala Ser Leu Pro Lys
   965 970 975
   Ser Met Glu Gly Tyr Tyr Gln Glu Ser Gly Arg Ala Gly Arg Asp Gly
   980 985 990
   Glu Ile Ser His Cys Val Leu Phe Tyr Thr Tyr His Asp Val Thr Arg
   995 1000 1005
   Leu Lys Arg Leu Ile Met Met Glu Lys Asp Gly Asn Tyr His Thr
   1010 1015 1020
   Lys Glu Thr His Val Asn Asn Leu Tyr Ser Met Val His Tyr Cys
   1025 1030 1035
   Glu Asn Ile Thr Glu Cys Arg Arg Ile Gln Leu Leu Ala Tyr Phe
   1040 1045 1050
   Gly Glu Lys Gly Phe Asn Pro Asp Phe Cys Lys Lys Tyr Pro Asp
   1055 1060 1065
   Val Ser Cys Asp Asn Cys Cys Lys Thr Lys Asp Tyr Lys Thr Lys
   1070 1075 1080
   Asp Val Thr Asp Asp Val Lys Asn Ile Ile Arg Phe Val Gln Glu
   1085 1090 1095
   His Ser Ser Ser Pro Gly Thr Arg Asn Ile Gly Pro Ala Gly Arg
   1100 1105 1110
   Phe Thr Leu Asn Met Leu Val Asp Ile Phe Leu Gly Ser Lys Ser
   1115 1120 1125
   Ala Lys Val Lys Ser Gly Ile Phe Gly Lys Gly Thr Thr Tyr Ser
   1130 1135 1140
   Arg His Asn Ala Glu Arg Leu Phe Lys Lys Leu Ile Leu Asp Lys
   1145 1150 1155
   Ile Leu Asp Glu Asp Leu Tyr Ile Asn Ala Asn Asp Gln Pro Ile
   1160 1165 1170
   Ala Tyr Val Met Leu Gly Thr Lys Ala His Ser Val Leu Ser Gly
   1175 1180 1185
   His Leu Lys Val Asp Phe Met Glu Thr Glu Asn Ser Ser Ser Ile
   1190 1195 1200
   Lys Lys Gln Lys Ala Leu Val Ala Lys Val Ser Gln Arg Glu Glu
   1205 1210 1215
   Val Val Lys Lys Cys Leu Gly Glu Leu Thr Glu Val Cys Lys Leu
   1220 1225 1230
   Leu Gly Lys Val Phe Gly Val His Tyr Phe Asn Ile Phe Asn Thr
   1235 1240 1245
   Ala Thr Leu Lys Lys Leu Ala Glu Ser Leu Ser Ser Asp Pro Glu
   1250 1255 1260
   Val Leu Leu Gln Ile Asp Gly Val Thr Glu Asp Lys Leu Glu Lys
   1265 1270 1275
   Tyr Gly Ala Glu Val Ile Pro Val Leu Gln Lys Tyr Ser Glu Trp
   1280 1285 1290
   Thr Val Pro Ala Glu Asp Gly Ser Pro Gly Ala Arg Gly Ala Pro
   1295 1300 1305
   Glu Asp  Thr Glu Glu Glu Glu  Glu Glu Ala Pro Val  Ser Ser His
       1310                 1315                 1320
   Tyr Phe  Ala Asn Gln Thr Arg  Asn Glu Arg Lys Arg  Lys Lys Met
       1325                 1330                 1335
   Ser Ala  Thr His Lys Pro Lys  Arg Arg Arg Thr Ser  Tyr Gly Gly
       1340                 1345                 1350
   Phe Arg  Ala Lys Gly Gly Ser  Thr Thr Cys Arg Lys  Thr Thr Ser
       1355                 1360                 1365
   Lys Ser  Lys Phe Tyr Gly Val  Thr Gly Ser Arg Ser  Ala Ser Cys
       1370                 1375                 1380
   Ala Ser  Gln Ala Thr Ser Ser  Ala Ser Arg Lys Leu  Gly Ile Met
       1385                 1390                 1395
   Ala Pro  Pro Lys Pro Val Asn  Arg Thr Phe Leu Arg  Pro Ser Tyr
       1400                 1405                 1410
   Ala Phe  Ser
       1415
<210> 3
   <211> 5315
   <212> DNA
   <213> Artificial
<220>
   <223> tet-off cassette sequence
<400> 3
   ccaagctggc catactggcc tgactagttc tagaattcgc tgtctgcgag ggccagctgt      60
   tggggtgagt actccctctc aaaagcgggc atgacttctg cgctaagatt gtcagtttcc     120
   aaaaacgagg aggatttgat attcacctgg cccgcggtga tgcctttgag ggtggccgcg     180
   tccatctggt cagaaaagac aatctttttg ttgtcaagct tgaggtgtgg caggcttgag     240
   atctggccat acacttgagt gacaatgaca tccactttgc ctttctctcc acaggtgtcc     300
   actcccaggt ccaactgcag cccaagcgga tcctgcggat aaccaccatg tctagattag     360
   ataaaagtaa agtgattaac agcgcattag agctgcttaa tgaggtcgga atcgaaggtt     420
   taacaacccg taaactcgcc cagaagctag gtgtagagca gcctacattg tattggcatg     480
   taaaaaataa gcgggctttg ctcgacgcct tagccattga gatgttagat aggcaccata     540
   ctcacttttg ccctttagaa ggggaaagct ggcaagattt tttacgtaat aacgctaaaa 600
   gttttagatg tgctttacta agtcatcgcg atggagcaaa agtacattta ggtacacggc 660
   ctacagaaaa acagtatgaa actctcgaaa atcaattagc ctttttatgc caacaaggtt 720
   tttcactaga gaatgcatta tatgcactca gcgctgtggg gcattttact ttgggttgcg 780
   tattggaaga tcaagagcat caagtcgcta aagaagaaag ggaaacacct actactgata 840
   gtatgccgcc attattacga caagctatcg aattatttga tcaccaaggt gcagagccag 900
   ccttcttatt cggccttgaa ttgatcatat gcggattaga aaaacaactt aaatgtgaaa 960
   gtgggtccgc gtacagccgc gcgcgtacga aaaacaatta cgggtctacc atcgagggcc 1020
   tgctcgatct cccggacgac gacgcccccg aagaggcggg gctggcggct ccgcgcctgt 1080
   cctttctccc cgcgggacac acgcgcagac tgtcgacggc ccccccgacc gatgtcagcc 1140
   tgggggacga gctccactta gacggcgagg acgtggcgat ggcgcatgcc gacgcgctag 1200
   acgatttcga tctggacatg ttgggggacg gggattcccc gggtccggga tttacccccc 1260
   acgactccgc cccctacggc gctctggata tggccgactt cgagtttgag cagatgttta 1320
   ccgatgccct tggaattgac gagtacggtg ggtagggggc gcgaggatcc agacatgata 1380
   agatacattg atgagtttgg acaaaccaca actagaatgc agtgaaaaaa atgctttatt 1440
   tgtgaaattt gtgatgctat tgctttattt gtaaccatta taagctgcaa taaacaagtt 1500
   aacaacaaca attgcattca ttttatgttt caggttcagg gggaggtgtg ggaggttttt 1560
   taaagcaagt aaaacctcta caaatgtggt atggctgatt atgatcctgc aagcctcgtc 1620
   gtcctggccg gaccacgcta tctgtgcaag gtccccggcc ccggacgcgc gctccatgag 1680
   cagagcgccc gccgccgagg cgaagactcg ggcggcgccc tgcccgtccc accaggtcaa 1740
   caggcggtaa cgtaaccggc ctcttcatcg ggaatgcgcg cgaccttcag catcgccggc 1800
   atgtccccct ggcggacggg aagtatccag ctcgaccaag cttctcgagg aattccgatc 1860
   atattcaata acccttaata taacttcgta taatgtatgc tatacgaagt tattaggtct 1920
   gaagaggagt ttacgtccag ccaagctagc ttggctgcag gtcgagcagt gtggttttca 1980
   agaggaagca aaaagcctct ccacccaggc ctggaatgtt tccacccaat gtcgagcaaa 2040
   ccccgcccag cgtcttgtca ttggcgaatt cgaacacgca gatgcagtcg gggcggcgcg 2100
   gtcccaggtc cacttcacat attaaggtga cgcgtgtggc ctcgaacacc gagcgaccct 2160
   gcagccaata tgggatcggc cattgaacaa gatggattgc acgcaggttc tccggccgct 2220
   tgggtggaga ggctattcgg ctatgactgg gcacaacaga caatcggctg ctctgatgcc 2280
   gccgtgttcc ggctgtcagc gcaggggcgc ccggttcttt ttgtcaagac cgacctgtcc 2340
   ggtgccctga atgaactgca ggacgaggca gcgcggctat cgtggctggc cacgacgggc 2400
   gttccttgcg cagctgtgct cgacgttgtc actgaagcgg gaagggactg gctgctattg 2460
   ggcgaagtgc cggggcagga tctcctgtca tctcaccttg ctcctgccga gaaagtatcc 2520
   atcatggctg atgcaatgcg gcggctgcat acgcttgatc cggctacctg cccattcgac 2580
   caccaagcga aacatcgcat cgagcgagca cgtactcgga tggaagccgg tcttgtcgat 2640
   caggatgatc tggacgaaga gcatcagggg ctcgcgccag ccgaactgtt cgccaggctc 2700
   aaggcgcgca tgcccgacgg cgaggatctc gtcgtgaccc atggcgatgc ctgcttgccg 2760
   aatatcatgg tggaaaatgg ccgcttttct ggattcatcg actgtggccg gctgggtgtg 2820
   gcggaccgct atcaggacat agcgttggct acccgtgata ttgctgaaga gcttggcggc 2880
   gaatgggctg accgcttcct cgtgctttac ggtatcgccg ctcccgattc gcagcgcatc 2940
   gccttctatc gccttcttga cgagttcttc tgaggggatc ggccaataaa aagacagaat 3000
   aaaacgcacg ggtgttgggt cgtttgttcg gatcggccgc gtatcacgag gccagctttt 3060
   caattcatca tttttttttt attctttttt ttgatttcgg tttccttgaa atttttttga 3120
   ttcggtaatc tccgaacaga aggaagaacg aaggaaggag cacagactta gattggtata 3180
   tatacgcata tgtagtgttg aagaaacatg aaattgccca gctattctta acccaactgc 3240
   acagtaacaa aaacatgcag gaaacgaaga taaatcatgt cgaaagctac atataaggaa 3300
   cgtgctgcta ctcatcctag tcctgttgct gccaagctat ttaatatcat gcacgaaaag 3360
   caaacaaact tgtgtgcttc attggatgtt cgtaccacca aggaattact ggagttagtt 3420
   gaagcattag gtcccaaaat ttgtttacta aaaacacatg tggatatctt gactgatttt 3480
   tccatggagg gcacagttaa gccgctaaag gcattatccg ccaagtacaa ttttttactc 3540
   ttcgaagaca gaaaatttgc tgacattggt aatacagtca aattgcagta ctctgcgggt 3600
   gtatacagaa tagcagaatg ggcagacatt acgaatgcac acggtgtggt gggcccaggt 3660
   attgttagcg gtttgaagca ggcggcggaa gaagtaacaa aggaacctag aggccttttg 3720
   atgttagcag aattgtcatg caagggctcc ctatctactg gagaatatac taagggtact 3780
   gttgacattg cgaagagcga caaagatttt gttatcggct ttattgctca aagagacatg 3840
   ggtggaagag atgaaggtta cgattggttg attatgacac ccggtgtggg tttagatgac 3900
   aagggagacg cattgggtca acagtataga accgtggatg atgtggtctc tacaggatct 3960
   gacattatta ttgttggaag aggactattt gcaaagggaa gggatgctaa ggtagagggt 4020
   gaacgttaca gaaaagcagg ctgggaagca tatttgagaa gatgcggcca gcaaaactaa 4080
   aaaactgtat tataagtaaa tgcatgtata ctaaactcac aaattagagc ttcaatttaa 4140
   ttatatcagt tattacccgc ggccgatccg tcgaggaatt ccgatcatat tcaataaccc 4200
   ttaatataac ttcgtataat gtatgctata cgaagttatt aggtctgaag aggagtttac 4260
   gtccagccaa gctagcttgg ctgcaggtcg agtaccccgg gttcgaaatc gatagatctc 4320
   ccgggtggca tccctgtgac ccctccccag tgcctctcct ggccctggaa gttgccactc 4380
   cagtgcccac cagccttgtc ctaataaaat taagttgcat cattttgtct gactagggtg 4440
   tccttctata atattatggg gtggaggggg ggtggtatgg agcaaggggc aagttgggaa 4500
   gacacacctg tagggcctgc ggggtctatt gggaaccaag cttggagtgc agtggcacaa 4560
   tcttggctca ctgcaatctc cgcctcctgg gttcaagcga ttctcctgcc tcagcctccc 4620
   gagttgttgg gatttccagg catgcatgac caggctcagc taatttttgt ttttttggta 4680
   gagacggggt ttcaccatat tggccaggct ggtctccaac tcctaatctc aggtgatcta 4740
   cccaccttgg cctcccaaat tgctgggatt acaggcgtga accactgctc ccttccctgt 4800
   ccttctgatt ttaaaataac tataccagca ggaggacgtc cagacacagc ataggctacc 4860
   tgccatggcc caaccggtgg gacatttgag ttgcttgctt ggcactgtcc tctcatgcgt 4920
   tgggtccact cagtagatgc ctgttgaatt cggtaccgag ctcgactttc acttttctct 4980
   atcactgata gggagtggta aactcgactt tcacttttct ctatcactga tagggagtgg 5040
   taaactcgac tttcactttt ctctatcact gatagggagt ggtaaactcg actttcactt 5100
   ttctctatca ctgataggga gtggtaaact cgactttcac ttttctctat cactgatagg 5160
   gagtggtaaa ctcgacctat ataagcagag ctcgtttagt gaaccgtcag atcgcctgga 5220
   gacgccatcc acgctgtttt gacctccata gaagacaccg ggaccgatcc agcctccgcg 5280
   gccccgaatt cctgcactta agcctaggcg gccgc 5315
<210> 4
   <211> 804
   <212> DNA
   <213> Artificial
<220>
   <223> mutant neo gene
<220>
   <221> CDS
   <222> (1)..(804)
<400> 4
   atg gga tcg gcc att gaa caa gat gga ttg cac gca ggt tct ccg gcc      48
   Met Gly Ser Ala Ile Glu Gln Asp Gly Leu His Ala Gly Ser Pro Ala
   1               5                   10                  15
   gct tgg gtg gag agg cta ttc ggc tat gac tgg gca caa cag aca atc      96
   Ala Trp Val Glu Arg Leu Phe Gly Tyr Asp Trp Ala Gln Gln Thr Ile
               20                  25                  30
   ggc tgc tct gat gcc gcc gtg ttc cgg ctg tca gcg cag ggg cgc ccg        144
   Gly Cys Ser Asp Ala Ala Val Phe Arg Leu Ser Ala Gln Gly Arg Pro
           35                  40                  45
   gtt ctt ttt gtc aag acc gac ctg tcc ggt gcc ctg aat gaa ctg cag        192
   Val Leu Phe Val Lys Thr Asp Leu Ser Gly Ala Leu Asn Glu Leu Gln
       50                  55                  60
   gac gag gca gcg cgg cta tcg tgg ctg gcc acg acg ggc gtt cct tgc        240
   Asp Glu Ala Ala Arg Leu Ser Trp Leu Ala Thr Thr Gly Val Pro Cys
   65                  70                  75                  80
   gca gct gtg ctc gac gtt gtc act gaa gcg gga agg gac tgg ctg cta        288
   Ala Ala Val Leu Asp Val Val Thr Glu Ala Gly Arg Asp Trp Leu Leu
                   85                  90                  95
   ttg ggc gaa gtg ccg ggg cag gat ctc ctg tca tct cac ctt gct cct        336
   Leu Gly Glu Val Pro Gly Gln Asp Leu Leu Ser Ser His Leu Ala Pro
               100                 105                 110
   gcc gag aaa gta tcc atc atg gct gat gca atg cgg cgg ctg cat acg        384
   Ala Glu Lys Val Ser Ile Met Ala Asp Ala Met Arg Arg Leu His Thr
           115                 120                 125
   ctt gat ccg gct acc tgc cca ttc gac cac caa gcg aaa cat cgc atc        432
   Leu Asp Pro Ala Thr Cys Pro Phe Asp His Gln Ala Lys His Arg Ile
       130                 135                 140
   gag cga gca cgt act cgg atg gaa gcc ggt ctt gtc gat cag gat gat        480
   Glu Arg Ala Arg Thr Arg Met Glu Ala Gly Leu Val Asp Gln Asp Asp
   145                 150                 155                 160
   ctg gac gaa gag cat cag ggg ctc gcg cca gcc gaa ctg ttc gcc agg        528
   Leu Asp Glu Glu His Gln Gly Leu Ala Pro Ala Glu Leu Phe Ala Arg
                   165                 170                 175
   ctc aag gcg cgc atg ccc gac ggc gat gat ctc gtc gtg acc cat ggc        576
   Leu Lys Ala Arg Met Pro Asp Gly Asp Asp Leu Val Val Thr His Gly
               180                 185                 190
   gat gcc tgc ttg ccg aat atc atg gtg gaa aat ggc cgc ttt tct gga        624
   Asp Ala Cys Leu Pro Asn Ile Met Val Glu Asn Gly Arg Phe Ser Gly
           195                 200                 205
   ttc atc gac tgt ggc cgg ctg ggt gtg gcg gac cgc tat cag gac ata        672
   Phe Ile Asp Cys Gly Arg Leu Gly Val Ala Asp Arg Tyr Gln Asp Ile
       210                 215                 220
   gcg ttg gct acc cgt gat att gct gaa gag ctt ggc ggc gaa tgg gct        720
   Ala Leu Ala Thr Arg Asp Ile Ala Glu Glu Leu Gly Gly Glu Trp Ala
   225                 230                 235                 240
   gac cgc ttc ctc gtg ctt tac ggt atc gcc gct ccc gat tcg cag cgc        768
   Asp Arg Phe Leu Val Leu Tyr Gly Ile Ala Ala Pro Asp Ser Gln Arg
                   245                 250                 255
   atc gcc ttc tat cgc ctt ctt gac gag ttc ttc tga                        804
   Ile Ala Phe Tyr Arg Leu Leu Asp Glu Phe Phe
               260                 265
<210> 5
   <211> 267
   <212> PRT
   <213> Artificial
<220>
   <223> Synthetic Construct
<400> 5
   Met Gly Ser Ala Ile Glu Gln Asp Gly Leu His Ala Gly Ser Pro Ala
   1               5                   10                  15
   Ala Trp Val Glu Arg Leu Phe Gly Tyr Asp Trp Ala Gln Gln Thr Ile
               20                  25                  30
   Gly Cys Ser Asp Ala Ala Val Phe Arg Leu Ser Ala Gln Gly Arg Pro
           35                  40                  45
   Val Leu Phe Val Lys Thr Asp Leu Ser Gly Ala Leu Asn Glu Leu Gln
       50                  55                  60
   Asp Glu Ala Ala Arg Leu Ser Trp Leu Ala Thr Thr Gly Val Pro Cys
   65                  70                  75                  80
   Ala Ala Val Leu Asp Val Val Thr Glu Ala Gly Arg Asp Trp Leu Leu
                   85                  90                  95
   Leu Gly Glu Val Pro Gly Gln Asp Leu Leu Ser Ser His Leu Ala Pro
               100                 105                 110
   Ala Glu Lys Val Ser Ile Met Ala Asp Ala Met Arg Arg Leu His Thr
           115                 120                 125
   Leu Asp Pro Ala Thr Cys Pro Phe Asp His Gln Ala Lys His Arg Ile
       130                 135                 140
   Glu Arg Ala Arg Thr Arg Met Glu Ala Gly Leu Val Asp Gln Asp Asp
   145                 150                 155                 160
   Leu Asp Glu Glu His Gln Gly Leu Ala Pro Ala Glu Leu Phe Ala Arg
                   165                 170                 175
   Leu Lys Ala Arg Met Pro Asp Gly Asp Asp Leu Val Val Thr His Gly
               180                 185                 190
   Asp Ala Cys Leu Pro Asn Ile Met Val Glu Asn Gly Arg Phe Ser Gly
           195                 200                 205
   Phe Ile Asp Cys Gly Arg Leu Gly Val Ala Asp Arg Tyr Gln Asp Ile
       210                 215                 220
   Ala Leu Ala Thr Arg Asp Ile Ala Glu Glu Leu Gly Gly Glu Trp Ala
   225                 230                 235                 240
   Asp Arg Phe Leu Val Leu Tyr Gly Ile Ala Ala Pro Asp Ser Gln Arg
                   245                 250                 255
   Ile Ala Phe Tyr Arg Leu Leu Asp Glu Phe Phe
               260                 265

## Description

### TECHNICAL FIELD

The present invention relates to technology for modifying stem cells. More specifically, the present invention relates to a method for causing universal mutagenesis of a stem cell (for example, an embryonic stem cell), system thereof, and stem cells obtained thereby.

### BACKGROUND ART

Stem cells, which have pluripotency, such as embryonic stem cells (hereinafter also called "ES cells") have been of note, since ES cells can differentiate into various organs or tissues. For example, 'knock-out' mice can be generated from ES cells by means of gene targeting, and thus it utility is of note.

In particular, *in vitro* differentiation of ES cells to many different cell types such as haematopoietic cells, neurons and cardiomyocytes (see, for example, Kyba, M. and Daley, G. Q., Exp.Hematol.31:994-1006(2003), Kim, J. H. et al., Nature 418: 50-56 (2002); and Parisi, S. et al., J. Cell Biol. 163: 303-314 (2003) = Non-patent literatures 1-3) has been reported, suggesting the possibility of therapeutic applications (see, for example, Reubinoff, B. E., Pera, M. F., Fong, C. Y., Trouson, A. and Bongso, A., Nature Biotechnol. 18: 399-404 (2000); and Thomson, J. A. et al., Science 282: 1145-1147 (1998) = Non-patent literatures 4-5).

The generation of an ES cell library bearing mutations in both alleles (bi-allelic mutations) have been demanded as it is useful for analyzing the molecular mechanism of differentiation and the pluripotency of ES cells.
[Non-patent literature 1]
   Kyba, M. and Daley, G. Q., Exp. Hematol. 31: 994-1006 (2003)
[Non-patent literature 2]
   Kim, J. H. et al., Nature 418: 50-56(2002)
[Non-patent literature 3]
   Parisi, S. et al., J.Cell Biol.163:303-314(2003)
[Non-patent literature 4]
   Reubinoff, B. E., Pera, M. F., Fong, C. Y., Trouson, A. and Bongso, A., Nature Biotechnol. 18: 399-404(2000)
[Non-patent literature 5]
   Thomson, J. A. et al., Science 282: 1145-1147 (1998)

### DISCLOSURE OF INVENTION

### (PROBLEM TO BE SOLVED BY THE INVENTION)

The object of the present invention is to provide technology for providing stem cells having mutations on both alleles (biallelic genes).

### (MEANS FOR SOLVING THE PROBLEMS)

The present invention solves the above-mentioned problem by unexpectedly finding that conditional disruption of the Bloom's syndrome gene allows the introduction of mutations over the entire genome of cells such as ES cells.

Accordingly, the present invention provides the following:
In one aspect, the present invention provides a non-human stem cell with a modification incorporated into both strands of the alleles thereof.

In one embodiment, the stem cell is a non-human embryonic stem cell.

In one embodiment, the Bloom's syndrome (Blm) gene of the non-human stem cell has been deleted or modified such that the Blm gene does not function.

In one embodiment, the Bloom's syndrome gene comprises the sequence set forth in SEQ ID NO: 1 or a variant thereof.

In another aspect, the present invention provides a library of non-human stem cells with a modification incorporated into both strands of the allele thereof, wherein the stem cells included in the library have incorporated the modification over the entire genome thereof.

In this embodiment, the stem cells are non-human embryonic stem cells.

In this embodiment, the Bloom's syndrome (Blm) gene of the non-human stem cells has been deleted or modified such that the Blm gene does not function.

In this embodiment, the Bloom's syndrome gene comprises the sequence set forth in SEQ ID NO: 1 or a variant thereof.

In another aspect, the present invention provides a method for producing a non-human stem cell with a modification incorporated into both strands of the alleles thereof, the method comprising the steps of: A) providing a stem cell; B) making the the Bloom's syndrome gene in the stem cell unfunctional; and C) inducing mutation in the stem cell.

In this embodiment, the Bloom's syndrome gene is processed so as to be transiently dysfunctional.

In this embodiment, the Bloom's syndrome gene is processed so as to be transiently dysfunctional in the presence of an agent.

In this embodiment, the agent is selected from the group consisting of tetracycline, doxycyclin, estrogen derivatives and progesteron derivatives.

In this embodiment, the induction of mutation is selected from the group consisting of exposure to a mutagen, use of a transposon gene, exposure to ultraviolet and exposure to radioactive rays.

In this embodiment, the method further comprises the step of inducing homologous recombination.

In this embodiment, the method further comprises the step of inducing homologous recombination in the 4N phase of the cell, thereby inducing cell division after the induction.

In this embodiment, the non-human stem cell is an non-human embryonic stem cell.

In this embodiment, the non-human embryonic stem cell is a non-human mammalian embryonic stem cell.

In another aspect, the present invention provides a non-human stem cell obtained by a method according to the present invention.

In this embodiment, the inventive non-human stem cell is a non-human embryonic stem cell.

In another aspect, the present invention provides a tissue obtained by a non-human stem cell which is obtained by a method according to the present invention.

In another aspect, the present invention provides a biological non-human organism obtained by a non-human stem cell which is obtained by a method according to the present invention.

In another aspect, the present invention provides use of the Bloom's syndrome gene or a variant thereof for mutation of a non-human stem cell.

In this embodiment of the use of the present invention, the Bloom's syndrome gene is disrupoted or modified to be unfunctional in the stem cell. In a specific embodiment, the Bloom's syndrome gene comprises the sequence set forth in SEQ ID NO:1 or a variant thereof.

In another aspect, the present invention provides use of the Bloom's syndrome gene or a variant thereof for manufacturing a composition for mutating a non-human stem cell.

In this embodiment of the use for manufacturing a composition of the present invention, the Bloom's syndrome (Blm) gene has been modified such that the Blm gene does not function. In a specific embodiment, the Bloom's syndrome gene comprises the sequence set forth in SEQ ID NO: 1 or a variant thereof.

### (DETAILED DESCRIPTION OF THE INVENTION)

The chief limitation of phenotype-based genetic screening in mammalian systems is the diploid nature of the genome. Cells deficient in the Bloom's syndrome gene (Blm) show an increased rate of loss of heterozygosity (LOH) (see, German, J., Dermatol. Clin. 13: 7-18 (1995), Groden,J., Nakamura, Y. and German,J., Proc. Natl. Acad. Sci. USA 87:4315-4319 (1990) and Luo, G. et al., Nature Genet. 26: 424-429 (2000)).

In one specific embodiment, we have used a tetracycline-regulated Blm allele (Blm^{tet}) to introduce bi-allelic mutations across the genome in mouse embryonic stem (ES) cells. Transient loss of Blm expression induces homologous recombination not only between sister chromatids but also between homologous chromosomes. We considered that the phenotype of ES cells bearing bi-allelic mutations would be maintained after withdrawal of the tetracycline analogue doxycycline. Indeed, the combination of N-ethyl-N-nitrosourea (ENU) mutagenesis and transient loss of Blm expression enabled us to generate an ES cell library with genome-wide bi-allelic mutations. The library was evaluated by screening for mutants of glycosylphosphatidylinositol (GPI) -anchor biosynthesis, which involve at least 23 genes distributed throughout the genome. Mutants derived from 12 different genes were obtained and two unknown mutants were simultaneously isolated.

Thus, it is understood that these and other advantages of the present invention will be clear to those skilled in the art upon reading and understanding the following Detailed Description of the Invention in view of the appended drawings.

### (EFFECTS OF INVENTION)

The present invention provides efficient phenotype-based genetic screening and provides efficient technologies for identifying gene functions in non-human ES cells. The present invention also allows the phenotype-based analysis over the entire genome of non-human ES cells.

### BRIEF DESCRIPTION OF DRAWINGS

**[****FIGURE 1****]**

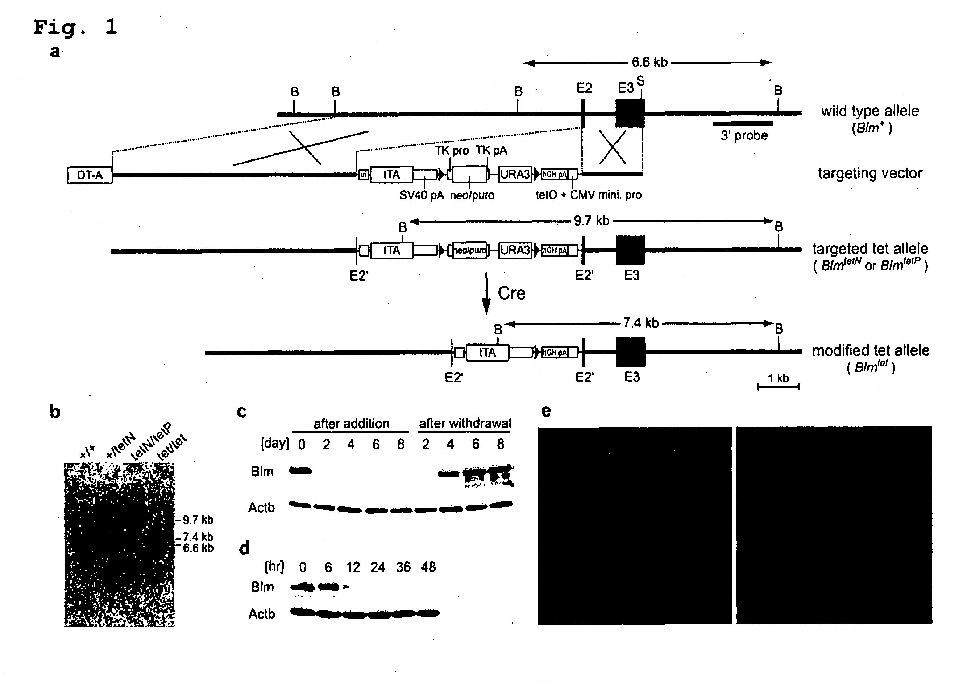

   **Figure 1** shows generation of conditional Blm alleles in ES cells and elevation of SCE. **Figure 1a** depicts the Targeting strategy and resulting Blm alleles. A tet cassette containing the neo or puro gene was inserted upstream of the translational initiation site of Blm to generate Blm^{tetN} or Blm^{tetP}, respectively. After Cre expression, these selection markers were deleted, resulting in Blm^{tet}. B, BamHI; S, SacI. Abbreviations for the tet cassette are described in Bond, C. T. et al., Science 289: 1942-1946(2000). **Figure 1b** depicts the Southern blot analysis of targeted clones. Genomic DNA was digested with BamHI, separated by electrophoresis and hybridized with the radiolabelled probe shown in Figure 1a. **Figures 1c** depicts the long-term analysis of Blm expression in Blm^{tet/tet} ES cells by western blot. Expression of β -actin (Actb) was used as a loading control. **Figures 1d** depicts the short-term analysis of Blm expression in Blm^{tet/tet} ES cells by western blot. Expression of b-actin (Actb) was used as a loading control. **Figure 1e** depicts the SCE of dox-treated (upper panel) or non-treated (lower panel) Blm^{tet/tet} ES cells.
**[****FIGURE 2****]**

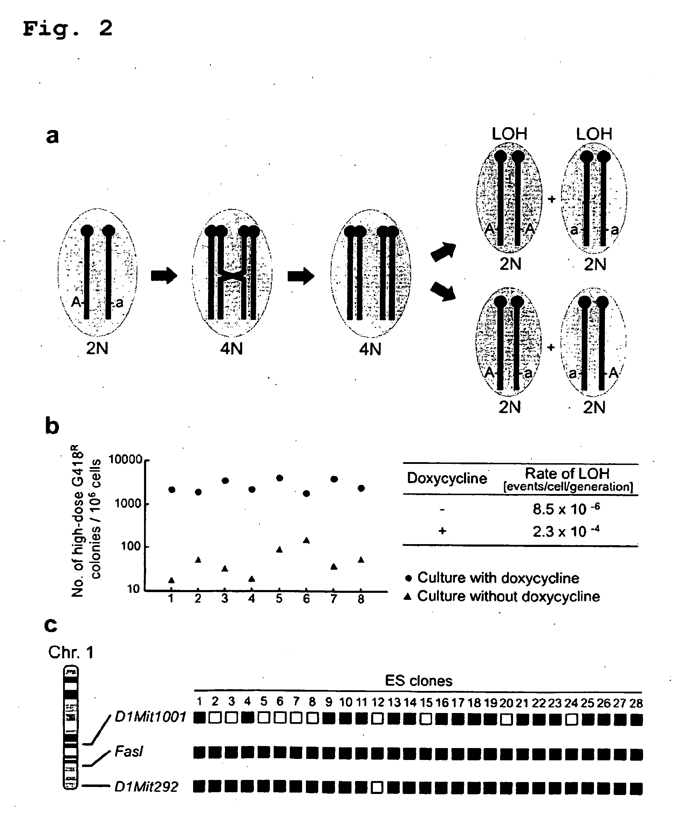

   **Figure 2** shows the high frequency of LOH in Blm-deficient ES cells. **Figure 2a** depicts a geneal scheme of mechanism of LOH. Heterozygosity is represented as A/a. When homologous recombination occurs at the 4N stage between homologous chromosomes, cells bearing LOH (A/A or a/a) appear after cell divisions. **Figure 2b** depicts a Luria-Delbruck fluctuation analysis of LOH. Frequency of duplication of the Fasl locus containing the neo gene was examined. c, Simple sequence length polymorphism (SSLP) marker analysis of 28 bi-allelic mutants of the Fasl locus. Open and filled squares indicate heterozygosity and LOH, respectively.
**[****FIGURE 3****]**

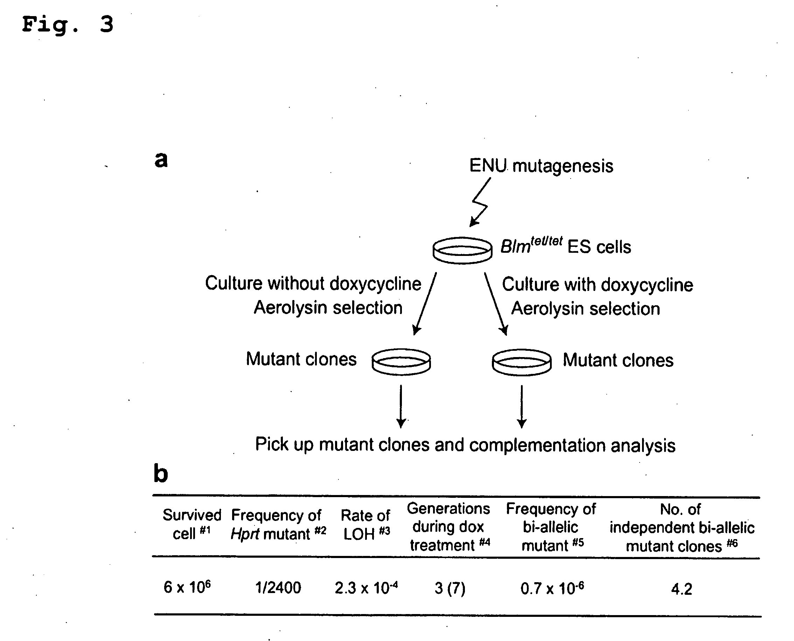

   **Figure 3** shows the construction of the mutant ES cell library and the screening strategy of GPI-anchord-efective mutants. **Figure 3a** depicts the screening of GPI-anchor-defective mutants from the ES library bearing bi-allelic mutations. **Figure 3b** depicts a theoretical prediction of the effectiveness of this screening strategy. From left to right, #1 depicts the number of surviving cells after ENU treatment of 2x10⁸ ES cells, #2 depicts the frequency of X-linked Hprt negative cells measured as a ratio of 6-TG resistant colonies, #3 depicts the mutation rate of LOH as described in **Figure 2b****,** #4 depicts the number of generations (cell cycles) during dox treatment (number in parentheses shows the total number of cell divisions during dox treatment), #5 depicts the frequency of clones bearing bi-allelic mutation per locus (2.3 x 10⁻⁴ x 1/2400 x 7), and #6, depicts the number of independent clones bearing bi-allelic mutation per locus after dox treatment.
**[****FIGURE 4****]**

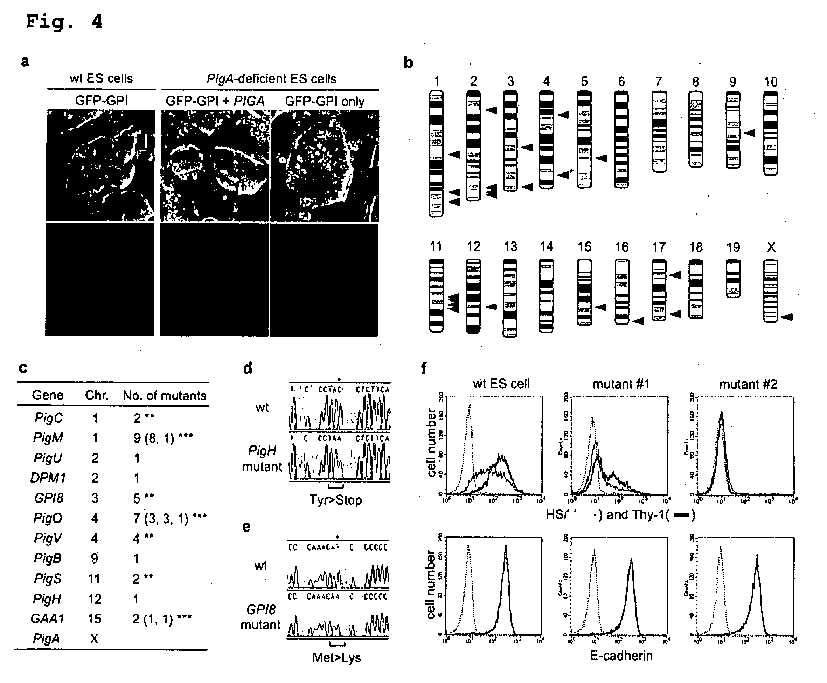

   **Figure 4** shows the analysis of GPI-anchor-defective mutants. **Figure 4a** depicts the complementation analysis of GPI-anchor-defective mutants. Right, GPI-anchored GFP proteins were expressed on the cell surface of PigA-deficient ES cells only when PigA cDNA was supplied. Left, wild-type ES cells were transfected with GFP-GPI as a positive control. **Figure 4b** depicts the chromosomal locations of 23 genes involved in GPI-anchor biosynthesis. Red arrowheads indicate 12 genes of which mutants were obtained in this screening; black arrowheads indicate 11 genes of which mutants were not obtained; asterisk indicates a cDNA that has been cloned but not published. **Figure 4c** depicts the chromosome location of mutated genes and the number of mutants obtained. The order of mutated genes in each chromosome is given from centromere to telomere. **Mutants containimg the same mutation; ***mutants containing different mutations. Numbers in parentheses indicate the number of mutants bearing the same mutation. **Figure 4d** depicts the sequence analysis of mutations in PigH. Homozygous mutations were verified. **Figure 4e** depicts the sequence analysis of mutations in GPI8. Homozygous mutations were verified. **Figure 4f** depicts the FACS analysis of two novel GPI mutants. Top, ES cells were stained with biotinylated anti-HSA (thin line) and anti-Thy-1 (thick line) antibodies, followed by streptavidinphycoerythrin. HSA and Thy-1 are GPI-anchored proteins. Broken lines (upper panel) indicate control staining profiles without biotinylated antibodies. Bottom, expression pattern of a non-GPIanchored protein, E-cadherin, in mutants.

### DESCRIPTION OF SEQUENCE LISTING

SEQ ID NO: 1 refers to the nucleic acid sequence of Bloom (Blm) gene;
SEQ ID NO: 2 refers to the amino acid sequence of Bloom (Blm) gene;
SEQ ID NO: 3 refers to the nucleic acid sequence of a cassette of the Blm allele, which is conditionally regulated by Tetracyclin, used in Example 1;
SEQ ID NO: 4 refers to the nucleic acid sequence of a neo gene mutant;
SEQ ID NO: 5 refers to the amino acid sequence of a neo gene mutant.

### BEST MODE FOR CARRYING OUT THE INVENTION

Hereinafter, the present invention will be described. It should be understood throughout the present specification that articles for a singular form (e.g., "a", "an", "the", etc. in English) include the concept of their plurality unless otherwise stated. It should be also understood that the terms as used herein have definitions as typically used in the art unless otherwise stated. Accordingly, unless otherwise defined, all technical and scientific terms used herein shall have the same meaning as that generally understood by those skilled in the art to which the present invention pertains. If there is any inconsistency, the present specification precedes, including definitions.

### (Definition of terms)

Hereinafter, terms specifically used herein will be defined.

The term "cell" is herein used in its broadest sense in the art, referring to a structural unit of the tissue of a multicellular organism, which is capable of self replicating, has genetic information and a mechanism for expressing it, and is surrounded by a membrane structure which isolates the cell from the outside. Cells used herein may be either naturally-occurring cells or artificially modified cells (e.g., fusion cells, genetically modified cells, etc.). Examples of cell sources include, but are not limited to, a single-cell culture; a non-human embryo, blood, or body tissue of normally-grown transgenic animals; a mixture of cells derived from normally-grown cell lines; and the like.

As used herein the term "4N phase" of a cell refers to a period of time in which the chromosomal number of a cell is duplicate in comparison to the normal state (2n). Such periods of time include but are not limited to: for example, G2 phase of the cellular cycle. Furthermore, it can be determined whether a cell is in the 4N phase or not by staining the chromosome thereof with propidium iodide (PI).

As used herein, the term "stem cell" refers to a cell capable of self replication and pluripotency. Typically, stem cells can regenerate an injured tissue. Stem cells used herein may be, but are not limited to, non-human embryonic stem (ES) cells or tissue stem cells (also called tissular stem cells, tissue-specific stem cells, or somatic stem cell). A stem cell may be an artificially produced cell (e.g., fusion cells, reprogrammed cells, or the like used herein) as long as it has the above-described abilities. Embryonic stem cells are pluripotent cells derived from early embryos. An embryonic stem cell was first established in 1981, which has been applied to the production of knockout mice since 1989. In 1998, a human embryonic stem cell was established, which is currently becoming available for regenerative medicine. Tissue stem cells have a relatively limited level of differentiation, unlike embryonic stem cells. Tissue stem cells are present in the particular place of tissues and have an undifferentiated intracellular structure. Therefore, tissue stem cells have a lower level of pluripotency. Tissue stem cells have a higher nucleus/cytoplasm ratio and have few intracellular organelles. Most tissue stem cells have pluripotency, a long cell cycle, and proliferative ability beyond the life of the individual. As used herein, stem cells may be preferably non-human embryonic stem cells, though tissue stem cells may also be employed, depending on the circumstance.

Tissue stem cells are separated into categories of sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, liver stem cells, and the like. Tissue stem cells in the bone marrow system include hematopoietic stem cells, mesenchymal stem cells, and the like. Tissue stem cells in the nervous system include neural stem cells, retinal stem cells, and the like.

As used herein, the term "somatic cell" refers to any cell other than a germ cell, such as an egg, a sperm, or the like, which does not transfer its DNA to the next generation. Typically, somatic cells have limited or no pluripotency.

The origin of a stem cell is categorized into the ectoderm, endoderm, or mesoderm. Stem cells of ectodermal origin are mostly present in the brain, including neural stem cells. Stem cells of mesoderm origin are mostly present in bone marrow, including blood vessel stem cells, hematopoietic stem cells, mesenchymal stem cells, and the like. Stem cells of endodermal origin are mostly present in organs, including liver stem cells, pancreas stem cells, and the like. Somatic cells may be herein derived from any germ layer.

As used herein, the term "isolated" means that naturally accompanying material is at least reduced, or preferably substantially completely eliminated, in normal circumstances. Therefore, the term "isolated cell" refers to a cell substantially free from other accompanying substances (e.g., other cells, proteins, nucleic acids, etc.) in natural circumstances. The term "isolated" in relation to nucleic acids or polypeptides means that, for example, the nucleic acids or the polypeptides are substantially free from cellular substances or culture media when they are produced by recombinant DNA techniques; or precursory chemical substances or other chemical substances when they are chemically synthesized. Isolated nucleic acids are preferably free from naturally flanking sequences within an organism from which the nucleic acids are derived (i.e., sequences positioned at the 5' terminus and the 3' terminus of the nucleic acids).

As used herein, the term "established" in relation to cells refers to a state in which a particular property (pluripotency) of the cell is maintained and the cell undergoes stable proliferation under culture conditions. Therefore, established stem cells maintain pluripotency. In the present invention, use of an established stem cell is preferable as the step of obtaining a new stem cell from a host can be avoided.

As used herein, "non-embryonic" refers to a state which is not directly derived from early embryo. Accordingly, cells derived from a portion of a body other than early embryo included therein, and in addition thereto, cells obtained by modification of an embryonic stem cells (for example, by genetic modification, fusion or the like), are also within the realm of non-embryonic cells.

As used herein, the term "differentiated cell" refers to a cell having a specialized function and form (e.g., muscle cells, neurons, etc.). Unlike stem cells, differentiated cells have no or little pluripotency. Examples of differentiated cells include epidermial cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells, and the like.

As used herein, the terms "differentiation" or "cell differentiation" refers to the phenomenon where two or more types of cells having qualitative differences in form and/or function occur in a daughter cell population derived from the division of a single cell. Therefore, "differentiation" includes the process during which a population (family tree) of cells, which do not originally have a specific detectable feature, acquire a feature, such as the production of a specific protein, or the like. At present, cell differentiation is generally considered to be the state of a cell in which a specific group of genes in the genome are expressed. Cell differentiation can be identified by searching for intracellular or extracellular agents or conditions which elicit the above-described state of gene expression. Differentiated cells are stable in principle. Particularly, animal cells which have been differentiated once rarely re-differentiate into other types of cells.

As used herein, the term "pluripotency" refers to the nature of a cell, i.e., an ability to differentiate into one or more, preferably two or more, tissues or organs. Therefore, the terms "pluripotent" and "undifferentiated" are herein used interchangeably unless otherwise mentioned. Typically, the pluripotency of a cell is limited during development, and in an adult, cells constituting a tissue or organ rarely differentiate into different cells, that is, the pluripotency is usually lost. Particularly, epithelial cells resist to differentiate into other types of epithelial cells. Such differentiation typically occurs in pathological conditions, and is called metaplasia. However, mesenchymal cells tend to easily undergo metaplasia, i.e., alter to other mesenchymal cells, with a relatively simple stimuli. Therefore, mesenchymal cells have a high level of pluripotency. Embryonic stem cells have pluripotency. Tissue stem cells have pluripotency. As used herein, the ability to differentiate in to all types of cells constituting a living organism, such as a fertilized egg, is called "totipotency", and the term "pluripotency" may include the concept of totipotency. An example of an *in vitro* assay for determining whether or not a cell has pluripotency, includes, but is not limited to, culturing under conditions for inducing the formation and differentiation of embryoid bodies. Examples of an *in vivo* assay for determining the presence or absence of pluripotency, include, but are not limited to, implantation of a cell into an immunodeficient mouse so as to form teratoma, injection of a cell into a blastocyst so as to form a chimeric embryo, implantation of a cell into a tissue of an organism (e.g., injection of a cell into ascites) so as to undergo proliferation, and the like.

As used herein, the term "organ" refers to a morphologically independent structure, localized to a particular portion of an individual organism, in which a certain function is performed. In multicellular organisms (e.g., animals, plants), an organ consists of several tissues spatially arranged in a particular manner, each tissue being composed of a number of cells. An example of such an organ includes an organ relating to the vascular system. In one embodiment, organs targeted by the present invention include, but are not limited to, skin, blood vessels, cornea, kidney, heart, liver, umbilical cord, intestine, nerve, lung, placenta, pancreas, brain, peripheral limbs, retina, and the like. As used herein, cells differentiated from a pluripotent cell of the present invention include, but are not limited to: epidermal cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells, and the like.

As used herein, the term "tissue" refers to an aggregate of cells having substantially the same function and/or form in a multicellular organism. "Tissue" is typically an aggregate of cells of the same origin, but may be an aggregate of cells of different origins as long as the cells have the same function and/or form. Therefore, when stem cells of the present invention are used to regenerate tissue, the tissue may be composed of an aggregate of cells of two or more different origins. Typically, a tissue constitutes a part of an organ. Animal tissues are separated into epithelial tissues, connective tissues, muscular tissues, nervous tissues, and the like, on a morphological, functional, or developmental basis. Plant tissues are roughly separated into meristematic tissues and permanent tissues, according to the developmental stage of the cells constituting the tissue. Alternatively, tissues may be separated into single tissues and composite tissues according to the type of cells constituting the tissue. Thus, tissues are separated into various categories.

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or nonnaturally-occurring amino acid, or a variant amino acid. The term may include those assembled into a composite of a plurality of polypeptide chains. The term also includes naturally-occurring or artificially modified amino acid polymers. Such modification includes, for example, disulfide bond formation, glycosylation, lipidation, acetylation, phosphorylation, or any other manipulation or modification (e.g., conjugation with a labeling moiety). This definition encompasses a polypeptide containing at least one amino acid analog (e.g., nonnaturally-occurring amino acid, etc.), a peptide-like compound (e.g., peptoid), and other variants known in the art, for example.

The terms "polynucleotide", "oligonucleotide", and "nucleic acid" as used herein have the same meaning and refer to a nucleotide polymer having any length. This term also includes an "oligonucleotide derivative" or a "polynucleotide derivative". An "oligonucleotide derivative" or a "polynucleotide derivative" includes a nucleotide derivative, or refers to an oligonucleotide or a polynucleotide having different linkages between nucleotides from typical linkages, which are interchangeably used. Examples of such oligonucleotides specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond are converted to a peptide-nucleic acid bond, an oligonucleotide derivative in which uracil is substituted with C-5 propynyl uracil, an oligonucleotide derivative in which uracil is substituted with C-5 thiazole uracil, an oligonucleotide derivative in which cytosine is substituted with C-5 propynyl cytosine, an oligonucleotide derivative in which cytosine is substituted with phenoxazine-modified cytosine, an oligonucleotide derivative in which ribose is substituted with 2'-O-propyl ribose, and an oligonucleotide derivative in which ribose is substituted with 2'-methoxyethoxy ribose. Unless otherwise indicated, particular nucleic acid sequences also implicitly encompasses conservatively-modified variants thereof (e.g. degenerate codon substitutions) and complementary sequences as well as sequences explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)).

As used herein the term "nucleic acid molecule" is also used interchangeably with nucleic acid, oligonucleotide, and polynucleotide, and includes cDNA, mRNA, genomic DNA and the like. As used herein, the nucleic acid and the nucleic acid molecule may be within the concept of the term "gene".

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene (e.g., promoter). Genes herein include structural genes and regulatory genes unless otherwise specified. Therefore, the term "Bloom's syndrome (Blm) gene" typically includes the structural gene of Bloom's syndrome (Blm) and the promoter of Bloom's syndrome (Blm). As used herein, "gene" may refer to "polynucleotide", "oligonucleotide" and "nucleic acid", and/or "protein", "polypeptide", "oligopeptide" and "peptide". As used herein, "gene product" includes "polynucleotide", "oligonucleotide" and "nucleic acid" and/or "protein", "polypeptide", "oligopeptide" and "peptide", which are expressed by a gene. Those skilled in the art understand what a gene product is, according to the context.

As used herein, the term "homology" in relation to a sequence (e.g., a nucleic acid sequence, an amino acid sequence, etc.) refers to the level of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other. As used herein, the term "similarity" in relation to a sequence (e.g., a nucleic acid sequence, an amino acid sequence, or the like) refers to the level of identity between two or more sequences when conservative substitution is regarded as positive (identical) in the above-described homology. Therefore, homology and similarity differ from each other in the presence of conservative substitutions. If no conservative substitutions are present, homology and similarity have the same value.

As used herein, the comparison of similarity, identity and homology of an amino acid sequence and a nuleotide sequence is calculated with BLAST, a tool for sequence analysis using default parameters.

As used herein, the term "amino acid" may refer to a naturally-occurring or nonnaturally-occurring amino acid as long as it satisfies the purpose of the present invention. The term "amino acid derivative" or "amino acid analog" refers to an amino acid which is different from a naturally-occurring amino acid and has a function similar to that of the original amino acid. Such amino acid derivatives and amino acid analogs are well known in the art.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the term "corresponding" amino acid or nucleic acid refers to an amino acid or nucleotide in a given polypeptide or polynucleotide molecule, which has, or is anticipated to have, a function similar to that of the predetermined amino acid or nucleotide in a polypeptide or polynucleotide as a reference for comparison. Particularly, in the case of enzyme molecules, the term refers to an amino acid which is present at a similar position in an active site and similarly contributes to its catalytic activity. For example, in the case of antisense molecules in a given polynucleotide, the term refers to a similar portion in an ortholog corresponding to a particular portion of the antisense molecule. As used herein, it should be understood that with respect to the amino acids responsible for functions of the Bloom's syndrome gene, amino acids corresponding to the other animals to the murine Bloom's syndrome gene are also responsible for such functions.

As used herein, the term "corresponding" gene refers to a gene in a given species, which has, or is anticipated to have, a function similar to that of a predetermined gene in a species as the reference for comparison. When there are a plurality of genes having such a function, the term refers to a gene having the same evolutionary origin. Therefore, a gene corresponding to a given gene may be an ortholog/a species homolog of the given gene. Therefore, genes corresponding to a mouse Bloom's syndrome gene and the like can be found in other animals. Such a corresponding gene can be identified by techniques well known in the art. Therefore, for example, a corresponding gene in a given animal can be found by searching in the sequence database of the animal (e.g., human, rat) using the sequence of a reference gene (e.g., mouse Bloom's syndrome genes, and the like) as a query sequence.

As used herein, the term "nucleotide" may be naturally-ocurring or not. "Nucleotide derivative" or "nucleotide analog" are interchangeably used herein to refer to a derivative or an analog which is different from a naturally occurring nucleotide but has a similar function as that of such a nucleotide. Such a nucleotide derivative and nucleotide analog is well known in the art. Examples of such a nucleotide derivative and nucleotide analog include, for example, but are not limited to phosphorothioate, phosphoramidate, methyl phosphonate, chiral methyl phosphonate, 2-O-methyl ribonucleotide, and peptide-nucleic acid (PNA).

As used herein, the term "fragment" with respect to a polypeptide or polynucleotide refers to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more amino acids. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. As used herein, the length of polypeptides or polynucleotides can be represented by the number of amino acids or nucleic acids, respectively. However, the above-described numbers are not absolute. The above-described numbers as the upper or lower limit are intended to include some greater or smaller numbers (e.g., ±10%), as long as the same function is maintained. For this purpose, "about" may be herein put ahead of the numbers. However, it should be understood that the interpretation of numbers is not affected by the presence or absence of "about" in the present specification.

As used herein the term "Bloom's syndrome gene", "Bloom gene", and "Blm gene" are interchangeably used to refer to a causative gene of syndrome by a genetic disorder related to DNA repair, which is a recessive genetic disease with microcephaly or dwarfism. Bloom Syndrom gene includes but is not limited to:
(A) nucleic acid molecules comprising:
   (a) a polynucleotide having a base sequence set forth in SEQ ID NO: 1 or a fragment sequence thereof;
   (b) a polynucleotide encoding a polypeptide consisting of an amino acid sequence set forth in SEQ ID NO: 2, or a fragment thereof;
   (c) a polynucleotide encoding a variant polypeptide consisting of an amino acid sequence set forth in SEQ ID NO: 2 with at least one mutation selected from at least one amino acid substitution, addition and deletion, or a fragment thereof, which possesses a biological activity;
   (d) a polynucleotide being a splice variant or allelic variant of the base sequence set forth in SEQ ID NO: 1, or a fragment thereof;
   (e) a polynucleotide encoding a species homolog of a polypeptide consisting of an amino acid sequence set forth in SEQ ID NO: 2, or a fragment thereof;
   (f) a polynucleotide which hybridizes to any of polynucleotides (a) through (e) or the complement thereof under stringent conditions, and encoding a polypeptide having a biological activity; or
   (g) a polynucleotide having at least 70 % identity to any of polynucleotides (a) through (e) or the complement thereof under stringent conditions, and encoding a polypeptide having a biological activity; or
(B) nucleic acid molecules encoding a polypeptide comprising:
   (a) a polypeptide encoded by a nucleic acid sequence as set forth in SEQ ID NO: 2 or a fragment thereof;
   (b) a polypeptide having the amino acid sequence as set forth in SEQ ID NO: 2 having at least one mutation selected from the group consisting of one or more amino acid substitutions, additions, and deletions, wherein the variant peptide has a biological activity;
   (c) a polypeptide encoded by a splice variant or allelic variant of the base sequence as set forth in SEQ ID NO: 1;
   (d) a species homolog polypeptide of a polypeptide having the amino acid sequence as set forth in SEQ ID NO: 2; or
   (e) a polypeptide consisting of an amino acid sequence having at least 70% identity to any one of the polypeptides of (a) to (d), having a biological activity.

As used herein the term "foreign gene" in a biological organism refers to a non naturally-occurring gene. Such a foreign gene may be a gene which has been modifed from a naturally-occurring gene in a biological organism, or a gene which is naturally occurring in another biological organism, such as Bloom gene, or a gene which has been artificially synthesized, or a complex (for example, a fusion) thereof. A biological organism comprising such a foreign gene may express a gene product which is not naturally expressed.

As used herein, the term "expression" of a gene product, such as a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

Accordingly, as used herein, "reduction" of "expression" of a gene, a polynucleotide, a polypeptide or the like refers to when the agent of the present invention is subjected to an action, and the amount of expression is significantly reduced compared to that when the agent is not subjected to an action. Preferably, the reduction of expression includes a reduction of the level of polypeptide expression. As used herein, the "increase" of "expression" of a gene, a polynucleotide, a polypeptide or the like refers to when the agent of the present invention is subjected to an action, resulting in an increase in the amount of expression as compared to when the agent is not subjected to an action. Preferably, the expression increase includes a subsequent increase in the level of the polypeptide expression. As used herein, the term "induction" of "expression" of a gene refers to an increase in the level of expression of the gene by acting an agent on a cell. Accordingly, the induction of expression encompasses the expression of the gene when no expression of the gene had been observed, and the increase in the level of expression of the gene when the level of the expression of the gene had already been observed.

As used herein the term "agent" refers to a compound having properties such as those in which a particular cell survive (or survive in a more potent manner), but other particular cells do not survive (or in a less potent manner). Cells may be selected by the presence and absence or strong and weak survival. Such an agent may include, but is not limited to, for example, tetracyclin, doxycyclin, estrogen derivatives and progensteron derivatives and the like. As used herein, such an agent is preferably a gene whose expression is induced or blocked by the presence of a foreign agent (for example, an antibiotic).

As used herein, term "biological activity" refers to activity prossessed by an agent (e.g., a polypeptide, a protein, etc.) within an organism, including activities exhibiting various functions (e.g., transcription promoting activity). For example, when a certain agent is an antisense molecule, the biological activity thereof is binding to the nucleic acid of interest, the expression inhibition thereby and the like. For example, when a certain agent is an enzyme, the biological activity thereof includes its enzymatic activity. In another example, when a certain agent is a ligand, the biological activity thereof includes the binding of the ligand to the corresponding receptor thereto. The above-described biological activity can be measured by techniques well-known in the art.

As used herein, the term "antisense (activity)" refers to activity which permits specific suppression or reduction of expression of a target gene. The antisense activity is ordinarily achieved by a nucleic acid sequence having a length of at least 8 contiguous nucleotides, which is complementary to the nucleic acid sequence of a target gene (for example, Blm). Such a nucleic acid sequence preferably has a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. These nucleic acid sequences include nucleic acid sequences having at least 70% homology thereto, more preferably at least 80%, even more preferably at least 90%, and still even more preferably at least 95%. The antisense activity is preferably complementary to a 5' terminal sequence of the nucleic acid sequence of a target gene. Such an antisense nucleic acid sequence includes the above-described sequences having one or several, or at least one, nucleotide substitutions, additions, and/or deletions.

As used herein, the term "RNAi" is an abbreviation of RNA interference and refers to a phenomenon where an agent for causing RNAi, such as double-stranded RNA (also called dsRNA), is introduced into cells and mRNA homologous thereto is specifically degraded, so that the synthesis of gene products is suppressed, and techniques using the phenomenon. As used herein, RNAi may have the same meaning as that of an agent which causes RNAi.

As used herein, the term "an agent causing RNAi" refers to any agent capable of causing RNAi. As used herein, "an agent causing RNAi of a gene" indicates that the agent causes RNAi relating to the gene and that the effect of RNAi is achieved (e.g., suppression of expression of the gene, and the like). Examples of such an agent causing RNAi include, but are not limited to, sequence having at least about 70% homology with the nucleic acid sequence of a target gene or a sequence hybridizable thereto under stringent conditions and RNA containing a double-stranded portion having a length of at least 10 nucleotides or variants thereof. Here, this agent may be preferably DNA containing a 3' protruding end, and more preferably the 3' protruding end has a length of 2 or more nucleotides (e.g., 2-4 nucleotides in length).

Though not wishing to be bound by any theory, a mechanism which causes RNAi is considered to be defined as follows. When a molecule which causes RNAi, such as dsRNA, is introduced into a cell, an RNaseIII-like nuclease having a helicase domain (called dicer) cleaves the molecule at about 20 base pair intervals from the 3' terminus in the presence of ATP in the case where the RNA is relatively long (e.g., 40 or more base pairs). As used herein, the term "siRNA" is an abbreviation of short interfering RNA and refers to short double-stranded RNA of 10 or more base pairs which are artificially chemically synthesized or biochemically synthesized, synthesized by an organism, or produced by double-stranded RNA of about 40 or more base pairs being degraded within the organism. siRNA typically has a structure comprising a 5'-phosphate and a 3'-OH, where the 3' terminus projects by about 2 bases. A specific protein is bound to siRNA to form RISC (RNA-induced-silencing-complex). This complex recognizes and binds to mRNA having the same sequence as that of siRNA and cleaves mRNA at the middle of siRNA due to RNaseIII-like enzymatic activity. It is preferable that the relationship between the sequence of siRNA and the sequence of mRNA to be cleaved as a target is a 100% match. However, base mutations at a site away from the middle of siRNA do not completely remove the cleavage activity by RNAi, leaving partial activity, while base mutations in the middle of siRNA have a large influence and the mRNA cleavage activity by RNAi is considerably lowered. By utilizing such a nature, only mRNA having a mutation can be specifically degraded. Specifically, siRNA in which the mutation is provided in the middle thereof is synthesized and is introduced into a cell. Therefore, in the present invention, siRNA per se, as well as an agent capable of producing siRNA (e.g., representatively dsRNA of about 40 or more base pairs) can be used as an agent capable of eliciting RNAi.

Also, though not wishing to be bound by any theory, apart from the above-described pathway, the antisense strand of siRNA binds to mRNA and siRNA functions as a primer for RNA-dependent RNA polymerase (RdRP), so that dsRNA is synthesized. This dsRNA is a substrate for the dicer again, leading to production of new siRNA. It is intended that such a reaction is amplified. Therefore, in the present invention, siRNA per se, as well as an agent capable of producing siRNA are useful. In fact, in insects and the like, for example, 35 dsRNA molecules can substantially completely degrade 1,000 or more copies of intracellular mRNA, and therefore, it will be understood that siRNA per se, as well as an agent capable of producing siRNA, is useful.

In the present invention, double-stranded RNA having a length of about 20 bases (e.g., representatively about 21 to 23 bases) or less than about 20 bases, called siRNA, can be used. Expression of siRNA in cells can suppress expression of a pathogenic gene targeted by the siRNA. Therefore, siRNA can be used for the treatment, prophylaxis, prognosis, and the like of diseases.

The siRNA of the present invention may be in any form as long as it can elicit RNAi.

In another embodiment, an agent capable of causing RNAi may have a short hairpin structure having a sticky portion at the 3' terminus (shRNA; short hairpin RNA). As used herein, the term "shRNA" refers to a molecule of about 20 or more base pairs in which a single-stranded RNA partially contains a palindromic base sequence and forms a double-strand structure therein (i.e., a hairpin structure). shRNA can be artificially chemically synthesized. Alternatively, shRNA can be produced by linking sense and antisense strands of a DNA sequence in reverse directions and synthesizing RNA *in vitro* with T7 RNA polymerase using the DNA as a template. Though not wishing to be bound by any theory, it should be understood that after shRNA is introduced into a cell, the shRNA is degraded in the cell to a length of about 20 bases (e.g., representatively 21, 22, 23 bases), and causes RNAi as with siRNA, leading to the treatment effects of the present invention. It should be understood that such an effect is exhibited in a wide range of organisms, such as insects, plants, animals (including mammals), and the like. Thus, shRNA elicits RNAi as with siRNA and therefore can be used as an effective component of the present invention. shRNA may preferably have a 3' protruding end. The length of the double-stranded portion is not particularly limited, but is preferably about 10 or more nucleotides, and more preferably about 20 or more nucleotides. Here, the 3' protruding end may be preferably DNA, more preferably DNA of at least 2 nucleotides in length, and even more preferably DNA of 2-4 nucleotides in length.

The agent capable of causing RNAi used in the present invention may be artificially synthesized (chemically or biochemically) or naturally occurring. There is substantially no difference in terms of the effect of the present invention. A chemically synthesized agent is preferably purified by liquid chromatography or the like.

The agent capable of causing RNAi used in the present invention can be produced *in vitro.* In this synthesis system, T7 RNA polymerase and T7 promoter are used to synthesize antisense and sense RNAs from template DNA. These RNAs are annealed and thereafter introduced into a cell. In this case, RNAi is caused via the above-described mechanism, thereby achieving the effect of the present invention. Here, for example, the introduction of RNA into cell can be carried out using a calcium phosphate method.

Another example of an agent capable of causing RNAi according to the present invention is a single-stranded nucleic acid hybridizable to mRNA, or all nucleic acid analogs thereof. Such agents are useful for the method and composition of the present invention.

As used herein, "polynucleotides hybridizing under stringent conditions" refers to conditions commonly used and well known in the art. Such polynucleotides can be obtained by conducting colony hybridization, plaque hybridization, Southern blot hybridization, or the like using a polynucleotide selected from the polynucleotides of the present invention. Specifically, a filter on which DNA derived from a colony or plaque is immobilized is used to conduct hybridization at 65°C in the presence of 0.7 to 1.0 M NaCl. Thereafter, a 0.1 to 2-fold concentration SSC (saline-sodium citrate) solution (1-fold concentration SSC solution composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A (adenine) or T (thymine). As used herein, "hybridizable polynucleotide" refers to a polynucleotide which can hybridize to other polynucleotides under the above-described hybridization conditions. Specifically, the hybridizable polynucleotide includes at least a polynucleotide having a homology of at least 60% to the base sequence of DNA encoding a polypeptide having an amino acid sequence as specifically set forth herein, preferably a polynucleotide having a homology of at least 80%, and more preferably a polynucleotide having a homology of at least 95%.

As used herein, the term "probe" refers to a substance for use in searching, which is used in a biological experiment, such as *in vitro* and/or *in vivo* screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90% or at least 95%.

As used herein, the term "search" indicates that a given nucleic acid sequence is utilized to find other nucleic acid base sequences having a specific function and/or property either electronically or biologically, or using other methods. Examples of an electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of a biological search include, but are not limited to, a macroarray in which genomic DNA is attached to a nylon membrane or the like or a microarray (microassay) in which genomic DNA is attached to a glass plate under stringent hybridization, PCR and in situ hybridization, and the like.

As used herein, the term "primer" refers to a substance required for the initiation of the reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, or at least 95%. An appropriate sequence as a primer may vary depending on the property of the sequence to be synthesized (amplified). Those skilled in the art can design an appropriate primer depending on the sequence of interest. Such a primer design is well known in the art and may be performed manually or using a computer program (e.g., LASERGENE, Primer Select, DNAStar).

As used herein, the term "agent binding specifically to" a certain nucleic acid molecule or polypeptide refers to an agent which has a level of binding to the nucleic acid molecule or polypeptide equal to or higher than a level of binding to other nucleic acid molecules or polypeptides. Examples of such an agent include, but are not limited to, when a target is a nucleic acid molecule, a nucleic acid molecule having a complementary sequence of a nucleic acid molecule of interest, a polypeptide capable of binding to a nucleic acid sequence of interest (e.g., a transcription agent, etc.), and the like, and when a target is a polypeptide, an antibody, a single chain antibody, either of a pair of a receptor and a ligand, either of a pair of an enzyme and a substrate, and the like.

### (Modification of genes)

When the above-described modifications are designed, the hydrophobicity indices of amino acids may be taken into consideration. Hydrophobic amino acid indices play an important role in providing a protein with an interactive biological function, which is generally recognized in the art (Kyte, J. and Doolittle, R.F., J. Mol. Biol. 157(1):105-132, 1982). The hydrophobic property of an amino acid contributes to the secondary structure of a protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0.5. It is understood in the art that such an amino acid substitution based on hydrophobicity is efficient.

The hydrophilicity index is also useful for the modification of an amino acid sequence of the present invention. As described in US Patent No. 4,554,101, amino acid residues are given the following hydrophilicity indices: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ± 1, and even more preferably ±0.5

The term "conservative substitution" as used herein refers to amino acid substitution in which a substituted amino acid and a substituting amino acid have similar hydrophilicity indices or/and hydrophobicity indices. For example, the conservative substitution is carried out between amino acids having a hydrophilicity or hydrophobicity index of within ±2, preferably within ±1, and more preferably within ±0.5. Examples of the conservative substitution include, but are not limited to, substitutions within each of the following residue pairs: arginine and lysine; glutamic acid and aspartic acid; serine and threonine; glutamine and asparagine; and valine, leucine, and isoleucine, which are well known to those skilled in the art.

As used herein, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. The term "allele" as used herein refers to a genetic variant located at a locus identical to a corresponding gene, where the two genes are distinguished from each other. Therefore, the term "allelic variant" as used herein refers to a variant which has an allelic relationship with a given gene. Such allelic variant ordinarily has a the same or a highly similar sequence to that of the corresponding allele, and ordinarily has almost the same biological activity, though it rarely has different biological activity. The term "species homolog" or "homolog" as used herein refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. The term "orthologs" (also called orthologous genes) refers to genes in different species derived from a common ancestry (due to speciation). For example, in the case of the hemoglobin gene family having multigene structure, human and mouse α-hemoglobin genes are orthologs, while the human α-hemoglobin gene and the human β-hemoglobin gene are paralogs (genes arising from gene duplication). Orthologs are useful for the estimation of molecular phylogenetic trees. Usually, orthologs in different species may have a function similar to that of the original species. Therefore, orthologs of the present invention may be useful in the present invention.

As used herein modification of Blm gene to render unfunctional may be achieved by modifying the normal function of the Blm gene by the disruption or reduction thereof, operating the cell not to function the Blm gene, rendering the Blm gene by means of an antisense or RNAi, or processing the cell by means of an agent which transiently dysfunction the gene, or the like.

As used herein the term "mutagenesis" refers to that a mutation is caused to a certain gene, and agents for inducing mutagenesis include but are not limited to: for example, mutagens (for example, N-ethyl-N-nitrosourea (ENU), nitrosoamin derivatives and the like), use of transposon gene, exposure to UV or radiation, and the like. It should be understood that those skilled in the art may practice mutagenesis using a transposon in view of the inventors' technology (see, WO 02/13602).

As used herein, the term "conservative (or conservatively modified) variant" applies to both amino acid and nucleic acid sequences. With respect to particular nucleic acid sequences, conservatively modified variants refer to nucleic acids encoding identical or essentially identical amino acid sequences. Because of the degeneracy of the genetic code, a large number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" which represent one species of conservatively modified variations. Every nucleic acid sequence herein which encodes a polypeptide also describes every possible silent variation of the nucleic acid. Those skilled in the art will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence. Preferably, such modification may be performed while avoiding substitution of cysteine which is an amino acid capable of largely affecting the higher-order structure of a polypeptide. Examples of methods for such modifications of a base sequence include cleavage using a restriction enzyme or the like; ligation or the like by treatment using DNA polymerase, Klenow fragments, DNA ligase, or the like; and a site specific base substitution method using synthesized oligonucleotides (specific-site directed mutagenesis; Mark Zoller and Michael Smith, Methods in Enzymology, 100, 468-500(1983)). Modification can be performed using methods ordinarily used in the field of molecular biology.

In order to prepare functionally equivalent polypeptides, amino acid additions, deletions, or modifications can be performed in addition to amino acid substitutions. Amino acid substitution(s) refers to the replacement of at least one amino acid of an original peptide with different amino acids, such as the replacement of 1 to 10 amino acids, preferably 1 to 5 amino acids, and more preferably 1 to 3 amino acids with different amino acids. Amino acid addition(s) refers to the addition of at least one amino acid to an original peptide chain, such as the addition of 1 to 10 amino acids, preferably 1 to 5 amino acids, and more preferably 1 to 3 amino acids to the original peptide chain. Amino acid deletion(s) refers to the deletion of at least one amino acid, such as the deletion of 1 to 10 amino acids, preferably 1 to 5 amino acids, and more preferably 1 to 3 amino acids. Amino acid modifications include, but are not limited to, amidation, carboxylation, sulfation, halogenation, alkylation, glycosylation, phosphorylation, hydroxylation, acylation (e.g., acetylation), and the like. Amino acids to be substituted or added may be naturally-occurring or nonnaturally-occurring amino acids, or amino acid analogs. Naturally-occurring amino acids are preferable.

As used herein, the term "peptide analog" or "peptide derivative" refers to a compound which is different from a peptide but has at least one chemical or biological function equivalent to the peptide. Therefore, a peptide analog includes one that has at least one amino acid analog or amino acid derivative addition or substitution with respect to the original peptide. A peptide analog has the above-described addition or substitution so that the function thereof is substantially the same as the function of the original peptide (e.g., a similar pKa value, a similar functional group, a similar binding manner to other molecules, a similar water-solubility, and the like). Such a peptide analog can be prepared using a technique well known in the art. Therefore, a peptide analog may be a polymer containing an amino acid analog.

Similarly, the term "polynucleotide analog" or "nucleic acid analog" refers to a compound which is different from the polynucleotide or nucleic acid but has at least one chemical function or biological function equivalent to that of the polynucleotide or nucleic acid. Therefore, polynucleotide analogs or nucleic acid analogs include one that has at least one nucleotide analog or nucleotide derivative addition or substitution with respect to the original peptide.

Nucleic acid molecules as used herein may include those in which a part of the sequence of the nucleic acid molecule is deleted or is substituted with other base(s), or an additional nucleic acid sequence is inserted, as long as the polypeptide expressed by the nucleic acid molecule has substantially the same activity as that of the naturally-occurring polypeptide, as described above. Alternatively, additional nucleic acids may be linked to the 5' terminus and/or 3' terminus of the nucleic acid molecule. The nucleic acid molecule may include one that is hybridizable to a gene encoding a polypeptide under stringent conditions and encodes a polypeptide having substantially the same function as that of that polypeptide. Such a gene is known in the art and can be used in the present invention.

The above-described nucleic acid molecule can be obtained by a well-known PCR method, i.e., chemical synthesis. This method may be combined with, for example, site-specific mutagenesis, hybridization, or the like.

As used herein, the term "substitution, addition or deletion" for a polypeptide or a polynucleotide refers to the substitution, addition or deletion of an amino acid or its substitute, or a nucleotide or its substitute with respect to the original polypeptide or polynucleotide sequence. This is achieved by techniques well known in the art, including a site-specific mutagenesis technique and the like. A polypeptide or a polynucleotide may have any number (>0) of substitutions, additions, or deletions. The number can be as large as the variant having such a number of substitutions, additions or deletions maintains the intended function (e.g., the information transfer function of hormones and cytokines, etc.). For example, such a number may be one or several, and preferably within 20% or 10% of the full length, or no more than 100, no more than 50, no more than 25, or the like.

As used herein, the term "specifically expressed" in the case of genes indicates that a gene is expressed in a specific site or in a specific period of time at a level different from (preferably higher than) that in other sites or periods of time. The term "specifically expressed" includes that a gene may be expressed only in a given site (specific site) or may be expressed in other sites. Preferably, the term "specifically expressed" indicates that a gene is expressed only in a given site.

As used herein the term "homologous recombination" refers to a crossover phenomenon in the DNA portions corresponding to each other between chromosomes. For example, such a homologous recombination is achieved by the deletion of Blm gene and using Cre/lox P system. In particular, when a homologous recombination is desired for inducing the 4N phase of the cell, it is achieved by subjection to the conditions of deletion of Blm gene and the use of Cre/loxP system.

Molecular biological, biochemical, and microorganism techniques as used herein are well known in the art and commonly used, and are described in, for example, Sambrook J. et al. (1989), Molecular Cloning: A Laboratory Manual, Cold Spring Harbor and its 3rd Ed. (2001); Ausubel, F.M. (1987), Current Protocols in Molecular Biology, Greene Pub. Associates and Wiley-Interscience; Ausubel, F.M. (1989), Short Protocols in Molecular Biology: A Compendium of Methods from Current Protocols in Molecular Biology, Greene Pub. Associates and Wiley-Interscience; Innis, M.A. (1990), PCR Protocols: A Guide to Methods and Applications, Academic Press; Ausubel, F.M. (1992), Short Protocols in Molecular Biology: A Compendium of Methods from Current Protocols in Molecular Biology, Greene Pub. Associates; Ausubel, F.M. (1995), Short Protocols in Molecular Biology: A Compendium of Methods from Current Protocols in Molecular Biology, Greene Pub. Associates; Innis, M.A. et al. (1995), PCR Strategies, Academic Press; Ausubel, F.M. (1999), Short Protocols in Molecular Biology: A Compendium of Methods from Current Protocols in Molecular Biology, Wiley, and annual updates; Sninsky, J.J. et al. (1999), PCR Applications: Protocols for Functional Genomics, Academic Press; Special issue, Jikken Igaku [Experimental Medicine] "Experimental Method for Gene Introduction & Expression Analysis", Yodo-sha, 1997; and the like. Relevant portions (or possibly the entirety) of each of these publication are herein incorporated by reference.

DNA synthesis techniques and nucleic acid chemistry for preparing artificially synthesized genes are described in, for example, Gait, M.J. (1985), Oligonucleotide Synthesis: A Practical Approach, IRL Press; Gait, M.J. (1990), Oligonucleotide Synthesis: A Practical Approach, IRL Press; Eckstein, F. (1991), Oligonucleotides and Analogues: A Practical Approac, IRL Press; Adams, R.L. et al. (1992), The Biochemistry of the Nucleic Acids, Chapman & Hall; Shabarova, Z. et al. (1994), Advanced Organic Chemistry of Nucleic Acids, Weinheim; Blackburn, G.M. et al. (1996), Nucleic Acids in Chemistry and Biology, Oxford University Press; Hermanson, G.T. (1996), Bioconjugate Techniques, Academic Press; and the like, related portions of which are herein incorporated by reference.

When a gene is mentioned herein, the term "vector" or "recombinant vector" refers to a vector capable of transferring the polynucleotide sequence of interest to a target cell. Such a vector is capable of self-replication or incorporation into a chromosome in a host cell (e.g., a prokaryotic cell, yeast, an animal cell, a plant cell, an insect cell, an individual animal, and an individual plant, etc.), and contains a promoter at a site suitable for transcription of the polynucleotide of the present invention. A vector suitable for cloning is referred to as "cloning vector". Such a cloning vector ordinarily contains a multiple cloning site containing a plurality of restriction sites. Presently, there are a number of vectors available for cloning a gene, which depend on a slight difference (for example, the types or sequence of a restriction enzyme of a multicloning site) from manufacturers. For example, typical sites and manufacturers thereof are described in Molecular Cloning (3rd edition) Sambrook, J and Russell, D.W., Appendix 3 (Volume 3), Vectors and Bacterial strains.A3.2 (Cold Spring Harbor USA,2001), and those skilled in the art may use such depending on the purpose of interest.

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes, and enhancers. It is well known to those skilled in the art that the type of an organism (e.g., an animal) expression vector and the type of a regulatory element may vary depending on the host cell.

Expression vectors used herein include, for example, lambda FIX vector (phage vector) for screening a genomic library, lambda ZAP vector (phage vector) for screening cDNA. pBluescript II SK+/-, pGEM, pCR2.1 vectors (plasmid vectors) can be typically used for cloning a genomic DNA. pSV2neo vector (plasmid vector) may be used as an expression vector. Such vectors may be practiced in view of Molecular Cloning A3.2 *supra.*

As used herein, the term "terminator" refers to a sequence which is located downstream of the protein-encoding region of a gene and which is involved in the termination of transcription when DNA is transcribed into mRNA, and the addition of a poly-A sequence. It is known that a terminator contributes to the stability of mRNA, and has an influence on the gene expression levels.

As used herein, the term "promoter" refers to a base sequence which determines the initiation site of gene transcription and is a DNA region which directly regulates the frequency of transcription. Transcription is started by RNA polymerase binding to the promoter. Accordingly, a portion having promoter function of a gene herein refers to "promoter moiety". The promoter region is usually located within about 2 kbp upstream of the first exon of a putative protein coding region. Therefore, it is possible to estimate a promoter region by predicting the protein coding region in a genomic base sequence using DNA analysis software. The putative promoter region is usually located upstream of a structural gene, but depending on the structural gene, i.e., downstream of a structural gene. Preferably, the putative promoter region is located within about 2 kbp upstream of the translation initiation site of the first exon.

As used herein, the term "enhancer" refers to a sequence which is used so as to enhance the expression efficiency of a gene of interest. Such enhancer is well known in the art.
One or more enhancers may be used, or no enhancer may be used.

As used herein, the term "operably linked" indicates that a desired sequence is located such that expression (operation) thereof is under control of a transcription and translation regulatory sequence (e.g., a promoter, an enhancer, and the like) or a translation regulatory sequence. In order for a promoter to be operably linked to a gene, typically, the promoter is located immediately upstream of the gene. A promoter is not necessarily adjacent to a structural gene.

As used herein the term "to process so as to transiently dysfunction" refers to the process in which a function of a certain gene is transiently made unfunctional, and includes for example, location of a gene so as to be unfunctional in the presence or absence of an agent (for example, a metal, antibiotic, and the like), which is responsible for on-off of a switch of the agent by operably linking a gene to another gene encoding the agent.

Any technique may be used herein for introduction of a nucleic acid molecule into cells, including, for example, transformation, transduction, transfection, and the like. Such a nucleic acid molecule introduction technique is well known in the art and commonly used, and is described in, for example, Ausubel F.A. et al., editors, (1988), Current Protocols in Molecular Biology, Wiley, New York, NY; Sambrook J. et al. (1987) Molecular Cloning: A Laboratory Manual, 2nd Ed. and its 3rd Ed., Cold Spring Harbor Laboratory Press, Cold Spring Harbor, NY; Special issue, Jikken Igaku [Experimental Medicine] "Experimental Method for Gene Introduction & Expression Analysis", Yodo-sha, 1997; and the like. Gene introduction can be confirmed by method as described herein, such as Northern blotting analysis and Western blotting analysis, or other well-known, common techniques.

Any of the above-described methods for introducing DNA into cells can be used as a vector introduction method, including, for example, transfection, transduction, transformation, and the like (e.g., a calcium phosphate method, a liposome method, a DEAE dextran method, an electroporation method, a particle gun (gene gun) method, and the like).

As used herein, the term "transformant" refers to the whole or a part of an organism, such as a cell, which is produced by transformation. Examples of a transformant include a prokaryotic cell, yeast, an animal cell, a plant cell, an insect cell, and the like. Transformants may be referred to as transformed cells, transformed tissue, transformed hosts, or the like, depending on the subject. A cell used herein may be a transformant.

When a prokaryotic cell is used herein for genetic operations or the like, the prokaryotic cell may be of, for example, genus *Escherichia,* genus *Serratia,* genus *Bacillus,* genus *Brevibacterium,* genus *Corynebacterium,* genus *Microbacterium,* genus *Pseudomonas*, or the like. Specifically, the prokaryotic cell is, for example, *Escherichia coli* XL1-Blue, *Escherichia coli* XL2-Blue, *Escherichia coli* DH1, or the like. Such cells are described in "Molecular Cloning (3rd edition)" by Sambrook, J and Russell, D.W., Appendix 3 (Volume 3), Vectors and Bacterial strains.A3.2 (Cold Spring Harbor USA 2001).

Animal cells which can be used in genetic engineering or the like herein, include murine myeloma cells, rat myeloma cells, murine hybridoma cells, Chinese Hamster cells including CHO cells, BHK cells, African green monkey kidney cells, human leukemia cells, HBT5637 (see Japanese Laid-Open Publication 63-299), human colon cancer cell line and the like. Murine myeloma cells include ps20, NSO and the like; rat myeloma cells include YB2/0 and the like; human fetal kidney cells include HEK293 (ATCC: CRL-1573) and the like; human leukemia cells include BALL-1 and the like; African green monkey kidney cells include COS-1, COS-7 and the like; human colon cancer cell lines include HCT-15. Preferably, for example, cells include but are not limited to COS-1, NIH3T3, ES (R1, TMA, NR2) cells and the like.

Any method for introduction of DNA can be used herein as a method for introduction of a recombinant vector, including, for example, a calcium chloride method, an electroporation method (Methods. Enzymol., 194, 182 (1990)), a lipofection method, a spheroplast method (Proc. Natl. Acad. Sci. USA, 84, 1929 (1978)), a lithium acetate method (J. Bacteriol., 153, 163 (1983)), (Proc. Natl. Acad. Sci. USA, 84, 1929 (1978) and the like.

The transient expression of Cre enzyme, DNA mapping on a chromosome, and the like, which are used herein in methods for removing a genome, a gene locus, or the like, are well known in the art, as described in Kenichi Matsubara and Hiroshi Yoshikawa, editors, Saibo-Kogaku [Cell Engineering], special issue, Experiment Protocol Series "FISH Experiment Protocol From Human Genome Analysis to Chrmosome/Gene diagnosis", Shujun-sha (Tokyo), and the like.

Gene expression (e.g., mRNA expression, polypeptide expression) may be "detected" or "quantified" by an appropriate method, including mRNA measurement and immunological measurement methods. Examples of molecular biological measurement methods include Northern blotting, dot blotting, PCR, and the like. Examples of immunological measurement methods include ELISA, RIA, fluorescent antibodies, Western blotting, immunohistological staining, and the like, where a microtiter plate may be used. Examples of quantification methods include ELISA, RIA, and the like. Gene analysis methods using arrays (e.g., a DNA array, a protein array, etc.) may be used. The DNA array is widely reviewed in Saibo-Kogaku [Cell Engineering], special issue, "DNA Microarray and Up-to-date PCR Method", edited by Shujun-sha. The protein array is described in detail in Nat Genet. 2002 Dec; 32 Suppl:526-32. Examples of methods for analyzing gene expression include, but are not limited to, RT-PCR, RACE, SSCP, immunoprecipitation, two-hybrid system, *in vitro* translation, and the like in addition to the above-described techniques. Other analysis methods are described in, for example, "Genome Analysis Experimental Method, Yusuke Nakamura's Labo-Manual, edited by Yusuke Nakamura, Yodo-sha (2002), and the like. All of the above-described publications are herein incorporated by reference.

### (Screening)

As used herein, the term "screening" refers to the selection of a target, such as an organism, a substance (for example, gene), or the like, or a given specific property of interest from a population containing a number of elements using a specific operation/evaluation method. For screening, the cell of the present invention may be used.

As used herein, screening by utilizing an immunological reaction is also referred to as "immunophenotyping". Various techniques employ monoclonal antibodies to screen for a cell population expressing a marker. Examples of such techniques include, but are not limited to, magnetic separation using magnetic beads coated with antibodies, "panning" using antibodies attached to a solid matrix (i.e., a plate), flow cytometry, and the like (e.g., US Patent No. 5,985,660; and Morrison et al., Cell, 96:737-49(1999)).

Screening may be performed using libraries obtained *in vitro, in vivo*, or the like (with a system using a real substance) or alternatively *in silico* (with a system using a computer). It will be understood that the present invention encompasses compounds having desired activity obtained by screening. The present invention is also intended to provide drugs, diagnositic agent and therapeutic agents which are produced by computer modeling based on the disclosures of the present invention.

As used herein the term "library" refers to a collection of genes, compounds, cells or the like for screening. Libraries may be a collection of genes, compounds, cells or the like having similar properties or random genes, compounds cells or the like. Preferably, collections of genes, compounds, cells or the like expected to have similar properties are used, but are not limited thereto.

Variants made according to the present invention (for example, ES cells), have been introduced to have modifications over the entire genome, and thus it is understood that the collection thereof may be used as a useful library for the analysis of a variety of genes.

It will also be understood that the patents, patent applications and literature cited herein should be incorporated by reference as if set forth fully herein as if the entirity were specifically described therein.

Hereinabove, the present invention has been described with preferable embodiments for ease of understanding. Hereinafter, the present invention will be described by way of examples. The examples below are provided only for illustrative purposes. Therefore, the scope of the present invention is limited only by the accompanying claims but not the examples.

### EXAMPLES

The animals used in the following examples have been cared for in accordance with the guidelines defined by Osaka University.

### (EXAMPLE 1: Generation of conditional Blm ES cells)

### (Method for generation of conditional Blm ES cells)

Genomic DNA containing the mouse Blm gene (SEQ ID NOS: 1 and 2) was isolated from the R1-ES genomic library. The targeting vector was introduced into R1-ES cells and selected by using G418 and/or puromycin (Sigma). Targeted clones were screened by polymerase chain reaction (Expand High Fidelity PCR System (Roche) and confirmed by Southern blot analysis (Rapid-hyb buffer (Amersham-Pharmacia). All targeted clones used in this study possessed a normal karyotype.

### (Western blot analysis)

Blm^{tet/tet}ES cells were cultured with 1.0 µg/ml of doxycycline (dox; Sigma) and collected at appropriate time points. To examine Blm expression after dox withdrawal, cells cultured in dox-containing media were washed once with PBS and further cultured in the absence of dox until collection. Blm protein was detected with an ab476 antibody directed against BLM (Abcam). For Western blotting analysis, ECL Western Blotting Detection Reagents (Amersham-Pharmacia) were used.

### (SCE analysis)

Blm^{tet/tet}ES cells were cultured in the presence or absence of dox for appropriate periods of time were labelled with 3 µg/ml of 5-bromodeoxyuridine (BrdU; Sigma) for 20 h and treated with 0.1 µg/ml of colcemid (KaryoMax-Colcemid (Invitrogen)) for 45 min. Chromosome spreads were stained with 0.1 mg/ml of acridine orange (Sigma).

### (Functional analysis)

Targeting vector for the Fasl locus with the mutant neo gene (see, Koike et al., EMBO Rep.3, 433-437 (2002); SEQ ID NOS: 4-5) was introduced into Blm^{tet/tet} ES cells. The rate of bi-allelic mutagenesis was measured by means of Luria-Delbruck functional analysis as described (see, Koike et al., EMBO Rep.3, 433-437 (2002)). In brief, Blm^{tet/tet}ES cells were cultured for 24 h with or without 1.0 µg/ml of dox and plated on a 100-mm dish with a clonal-density culture with or without dox to obtain single-cell clones. Expanded single-cell clones were then selected by using high-dose G418 (1.0 mg/ml) without dox. The number of high-dose G418 resistant clones was counted 10 days after selection.

### (ENU mutagenesis and screening for GPI-anchor mutants)

We carried out ENU mutagenesis and calculated the mutation frequency of the Hprt locus as described (Chen, Y. et al., Nature Genet. 24, 314-317 (2000)). The mutagenized ES cells were cultured with or without dox for 4 days to generate the mutant ES cell library. The mutant and control libraries were treated in 10 nM proaerolysin (Protox Biotech) in suspension (1.0 x 10⁶ cells per ml) and plated on a gelatin-coated 100-mm dish at 5-8 x 10⁶ cells per dish. The next day, dead cells were washed out and living cells were treated in 5 nM proaerolysin for 8 h before mitomycin-C (available from Kyowa Hakko KK). Treated feeder cells were added to generate GPI-anchor-defective mutant colonies. The resulting colonies were collected, expanded and transfected with GFP-GPI expression vector and cDNAs involved in GPI-anchor biosynthesis for the complementation assay (TransFast transfection reagent (Promega), DNA 2mg and TransFast (12µl) were added to 1.0×10⁵ ES cells).

### (RESULTS)

**Figure 1a** shows general scheme of the Blm allele under conditional regulation of tetracycline (Blm^{tet}) (SEQ ID NO: 3). Tetracycline-system-based regulatory cassettes (tet cassettes) (see, Bond, C. T. et al.,Science 289: 1942-1946 (2000)) were inserted immediately upstream of the translation initiation codons of both alleles of Blm to change them into Blm^{tet}. Targeting was confirmed by Southern blot analysis **(****Figure 1b****).** We considered that continuous deficiency of Blm would cause continuous accumulation of bi-allelic mutations in the genome, resulting in a change in phenotype during long-term culture, while transient loss of Blm caused by Blm^{tet} would minimize changes in the phenotype. Regulation of Blm expression was examined by using the tetracycline analogue, doxycycline (dox). Addition of dox resulted in a rapid reduction in Blm protein (**Figures 1c** and **1d****).** Notably, the Blm protein regained its original expression after the withdrawal of dox **(****Figure 1c****).** Increased numbers of sister chromatid exchanges (SCEs), a typical cytogenetic phenomenon of Bloom's syndrome cells (see, German, J., Dermatol.Clin. 13:7-18 (1995)), were observed **(****Figure 1e****),** while Blm proteins were undetectable **(****Figure 1c****).** SCE is closely coupled to homologous recombination in vertebrate cells (see, Sonoda, E. et al., Mol. Cell. Biol. 19; 5166-5169(1999)); therefore, dox treatment is expected to induce recombination between homologous chromosomes and, when it occurs at the 4N stage, a mono-allelic mutation will become bi-allelic after cell division **(****Figure 2a****).** To examine the effect of transient Blm deficiency on the rate of bi-allelic mutation, we introduced, by means of gene targeting, a mutant neo gene into the Fas ligand (Fasl) locus as a model of 'mono-allelic mutation'. We have previously shown that duplication of the mutant neo gene at this locus, which represents 'bi-allelic mutation', can be selected by high doses of G418 (see, Koike, H. et al., EMBO Rep.3: 433-437 (2002)). The rate of the duplication determined by Luria-Delbruck fluctuation analysis (see, Luria, S. E. and Delbruck, M., Genetics 28: 491-510 (1943)) was 8.5 x 10⁻⁶ events per cell per generation in the absence of dox, but increased to 2.3 x 10⁻⁴ events per cell per generation in the presence of dox **(****Figure 2b****).** Therefore, transient loss of Blm causes a 27-fold increase in the rate of bi-allelic mutation **(****Figure 2b****).** The rates obtained in this study are similar to those reported previously for loss of heterozygosity (LOH) in wildtype (2.3 x 10⁻⁵) and Blm-deficient (4.2 x 10⁻⁴) ES cells (see, Luo, G. et al., Nature Genet.26: 424-429 (2000)).

To determine the chromosomal locations of the crossover, we used polymorphic markers in chromosome 1 of the R1-ES cell line **(****Figure 2c****),** which was established from an F1 embryo obtained from the breeding of two different inbred 129 substrains (129X1/SvJ x 129S1/SvImJ) (see, Lefebvre, L., Dionne, N., Karaskova, J., Squire, J.A. and Nagy, A., Nature Genet. 27: 257-258 (2001)). The respective locations of the D1Mit1001 and D1Mit292 markers are located roughly 30 megabases (Mb) proximal and distal from the Fasl locus. Twenty-eight high-dose G418-resistant clones were chosen to determine the chromosomal locations of the crossover, and ten (ca. 35%) crossovers were found to occur in a region 30-Mb proximal from the Fasl locus **(****Figure 2c****).**

The observation of increased mitotic recombination in Blm-modified ES cells prompted us to examine the possibility of establishing an ES cell library containing the bi-allelic mutations throughout the genome. N-Ethyl-N-nitrosourea (ENU) was used as a mutagen because of its extremely high mutagenicity in ES cells (see, Chen, Y. et al., Nature Genet. 24: 314-317(2000)). To determine whether the distribution and complexity of bi-allelic mutations were high enough to cover the whole genome, we screened for mutant ES cells deficient in glycosylphosphatidylinositol (GPI)-anchor biosynthesis. At least 23 genes, which are widely distributed in the mouse genome, are involved in this pathway **(****Figure 4b****).** Because mutations in any gene in the GPI pathway yield a deficiency of GPI-anchored proteins on the cell surface, cells deficient in the GPI-anchor can be positively selected by using aerolysin, which kills cells with GPI-anchors (see, Hong,Y. et al., EMBO J. 21: 5047-5056 (2002)). The ES cell line used in this study was of male origin, whereas the PigA gene involved in the first step of GPI-anchor biosynthesis is localized on the X chromosome. Therefore, functional disruption of PigA does not require bi-allelic mutation and most of the GPI-anchor-deficient mutants would originate from PigA mutation (see, Kawagoe, K., Takeda, J., Endo, Y. and Kinoshita, T., Genomics 23: 566-574 (1994)). To avoid such a bias, extra copies of the PigA complementary DNA were introduced into the Blm^{tet/tet} ES cells before ENU mutagenesis.

**Figure 3a** summarizes the protocol for ENU mutagenesis of ES cells, generation of the ES cell library, and screening for GPI-anchor-defecient mutants. We treated 2 x 10⁸ ES cells using an ENU dose of 0.2 mg/ml for 2 h at 37 ° C. Cell viability was roughly 3%, resulting in 6 x 10⁶ cells surviving after ENU treatment. The mutation frequency in surviving cells was 1 in 2,400 at the X-linked monoallelic hypoxanthine phosphoribosyl transferase (Hprt) locus, as determined by selection with 6-thioguanine. It has been reported that ES cells treated at a higher concentration of ENU (0.35 mg/ml) retain germline competency (see, Chen, Y. et al., Nature Genet. 24: 314-317 (2000)), suggesting that the long-term viability of treated cells resembles that of wild-type ES cells. The frequency of bi-allelic mutations induced by dox treatment for three generations of cell cycles was calculated to be 0.7 x 10⁻⁶ per locus, and the number of independent clones bearing bi-allelic mutations was estimated at 4.2 per locus **(****Figure 3b****).** These results indicate that our ES cell library contains bi-allelic mutations in most loci.

To verify the practical utility of this principle, we screened the ES cell library with aerolysin, which resulted in the isolation of 35 GPI-anchor-deficient mutants **(****Figure 3a****).** When the GPI-anchored green fluorescent protein (GFP) construct (GFP-GPI) (see, Kondoh, G. et al., FEBS Lett. 458: 299-303(1999)) was transfected into wild-type ES cells, GFP-GPI proteins were expressed on the cell surface **(****Figure 4a****).** By contrast, GFP-GPI proteins were expressed on the mutants only when complementary cDNA was supplied **(****Figure 4a****).** These mutants were therefore classified by means of complementation analysis using the transfection of cDNAs of genes involved in GPI-anchor biosynthesis. The mutated genes were found to be distributed widely throughout the genome, and mutants were identified in more than half of the known GPI-anchor biosynthesis genes (12 out of 23) after one round of screening **(****Figure 4b). Figure 4c** shows that one mutant was obtained in four genes, but that more than one mutant was obtained in other autosomal genes. Because the genes that were affected frequently had same mutation, their mutants were probably derived from single clones. Therefore, the difference in mutant numbers would be explained by the differences in the stages at which the mutants were generated after the addition of dox. Sequence analysis of these mutants showed that they did not contain sequences of the wild-type allele **(****Figure 4d, e****),** indicating that bi-allelic mutations had occurred in the ES cells treated with dox. Without dox treatment, only one mutant was isolated (data not shown). In addition, GPI-anchor biosynthesis was not complemented by cDNA transfection in two mutants, which suggested that these mutants had mutations in novel genes involved in the GPI pathway **(****Figure 4f****).** Different genes were mutated in these mutants because the deficiency in GPI-anchor biosynthesis was complemented by cell fusion (data not shown).

We have shown that it is possible to isolate recessive mutants across the genome in mammalian cells. Although we (see, Koike, H. et al., EMBO Rep. 3: 433-437 (2002)) and others (see, Liu, P., Jenkins, N.A. and Copeland, N. G., Nature Genet. 30: 66-72 (2002) have reported a method to introduce bi-allelic mutation by Cre-loxP-mediated recombination between homologous chromosomes, that method can be applied only to a pre-selected chromosome that carries loxP sites on both alleles. Many, but not all, loci are functionally haploid in the Chinese hamster ovary (CHO) cell line (see, Gupta, R. S., Chan, D. Y. and Siminovitch, L., Cell 14: 1007-1013 (1978)), and the isolation of mutant cells with recessive phenotype has been reported (see, Hanada, K. et al., Nature 426: 803-809 (2003)). Because the probability of isolating mutants depends largely on whether target genes are functionally haploid or diploid in CHO cells, non-random isolation of mutants can be expected (see, Nakamura, N. et al., J. Biol. Chem. 272: 15834-15840 (1997)). In our study, more than half of the genes known to be involved in GPI-anchor biosynthesis could be identified in a single round of selection in ES cells with normal karyotype, thereby verifying the random nature of our selection scheme. Theoretically, bi-allelic mutations occur in most loci **(****Figure 3b****),** but nearly half of the genes involved in GPI-anchor biosynthesis could not be identified. This incomplete coverage may be explained by AT base-pair predominant mutations with ENU in ES cells (see, Munroe, R. J. et al., Nature Genet. 24: 318-321 (2000)). This possibility can be tested by other chemical mutagens such as EMS and ICR191, which have a different mutation spectrum.

To identify genes responsible for a given phenotype, expression cloning can be applied. The development of highly efficient systems for cDNA library transduction and recovery, such as an episomal vector stably maintained in ES cells (see, Chambers, I. et al., Cell 113: 643-655 (2003)) and high-titer retroviral vectors resistant to promoter silencing in ES cells (see, Kitamura, T. et al., Exp. Hematol. 31: 1007-1014 (2003)), will greatly aid the identification of mutated genes. A survey of homologous regions by means of polymorphic markers is likely to narrow down the location of the mutation, as exemplified by **Figure 2c****.** To achieve fine mapping of mutations, we introduced Blm^{tet} alleles into C57BL/6 x 129S4/SvJae F1 hybrid ES cells (see, Eggan, K. et al., Proc. Natl. Acad. Sci. USA 98: 6209-6214 (2001)), for which a large number of polymorphic markers are available. Alternatively, tagged mutagenesis such as gene trap (see, Friedrich, G. and Soriano, P., Genes Dev. 5: 1513-1523 (1991)) may be used in place of ENU to facilitate identification of causative genes. In fact, the retroviral genetrap vector has been used successfully in the accompanying paper for a genome-wide recessive screen.

For phenotype-based genetic screening, it is essential to establish definitive criteria for judging whether an observed phenotype is caused by genetic mutations or by a simple change in the characteristics of wild-type cells. This is especially important in the analysis of ES cells, because a small fraction of the ES cell population may differentiate spontaneously even when culture conditions are carefully controlled. Conditional regulation of Blm expression is useful in assessing a given phenotype. If clones with the desired phenotype can be obtained more efficiently under dox-treated than under nonselective culture conditions, then those clones isolated by dox-induced LOH probably contain bi-allelic mutations. Here, the fact that 35 aerolysin-resistant clones were obtained from dox-treated culture, as compared with only 1 clone with non-selective culture, prompted us to characterize those clones further. Because pluripotent ES cells can differentiate into any type of tissue, a method for the comprehensive isolation of bi-allelic mutants should have a major impact on the analysis of molecular mechanism of differentiation *in vitro* as well as *in vivo.*

### LIST OF REFERENCES

1. German, J. Dermatol. Clin. 13, 7-18 (1995).
2. Groden, J., Nakamura, Y. & German, J. Proc. Natl Acad. Sci. USA 87, 4315-4319 (1990).
3. Luo, G. et al. Nature Genet. 26, 424-429 (2000).
4. Kyba, M. & Daley, G. Q. Exp. Hematol. 31, 994-1006 (2003).
5. Kim, J. H. et al. Nature 418, 50-56 (2002).
6. Parisi, S. et al. J. Cell Biol. 163, 303-314 (2003).
7. Reubinoff, B. E., Pera, M. F., Fong, C. Y., Nature Biotechnol. 18, 399-404 (2000).
8. Thomson, J. A. et al. Science 282, 1145-1147 (1998).
9. Bond, C. T. et al. Science 289, 1942-1946 (2000).
10. Sonoda, E. et al. Mol. Cell. Biol. 19, 5166-5169 (1999).
11. Koike, H. et al. EMBO Rep. 3, 433-437 (2002).
12. Luria, S. E. & Delbruck, M. Genetics 28, 491-510 (1943).
13. Lefebvre, L., Dionne, N., Karaskova, J., Squire, J. A. & Nagy, A. Nature Genet. 27, 257-258 (2001).
14. Chen, Y. et al. Nature Genet. 24, 314-317 (2000).
15. Hong, Y. et al. EMBO J. 21, 5047-5056 (2002).
16. Kawagoe, K., Takeda, J., Endo, Y. & Kinoshita, T. Genomics 23, 566-574 (1994).
17. Kondoh, G. et al. FEBS Lett. 458, 299-303 (1999).
18. Liu, P., Jenkins, N. A. & Copeland, N. G. Nature Genet. 30, 66-72 (2002).
19. Gupta, R. S., Chan, D. Y. & Siminovitch, L. Cell 14, 1007-1013 (1978).
20. Hanada, K. et al. Nature 426, 803-809 (2003).
21. Nakamura, N. et al. J. Biol. Chem. 272, 15834-15840 (1997).
22. Munroe, R. J. et al. Nature Genet. 24, 318-321 (2000).
23. Chambers, I. et al. Cell 113, 643-655 (2003).
24. Kitamura, T. et al. Exp. Hematol. 31, 1007-1014 (2003).
25. Eggan, K. et al. Proc. Natl Acad. Sci. USA 98, 6209-6214 (2001).
26. Friedrich, G. & Soriano, P. Genes Dev. 5, 1513-1523 (1991).

Although certain preferred embodiments have been described herein, it is not intended that such embodiments be construed as limitations on the scope of the invention except as set forth in the appended claims.

### INDUSTRIAL APPLICABILITY

In the present invention, mutations may be universely induced to stem cells such as non-human ES cells, and the mutations may be introduced over the entire genome, and thus a stem cell library which may be used for analyzing the variety of genes is provided. This library may be used for developing pharmaceuticals, analyzing diseases, diagnosing diseases, therapy, gene therapy and the like, and thus highly industrially applicable.

## Claims

1. A non-human stem cell with a modification incorporated into both strands of the alleles thereof and wherein the Bloom's syndrome (Blm) gene has been deleted or modified such that the gene does not function and the cell shows increased loss of heterozygosity, wherein the Bloom's syndrome (Blm) gene comprises the sequence set forth in SEQ ID NO:1 or a variant thereof with at least 70% identity to SEQ ID No:1.

2. A non-human stem cell according to claim 1, which is an embryonic stem cell.

3. A stem cell according to claim 1, wherein said gene consists of a nucleotide sequence with at least 80% identity to the nucleotide sequence set forth in SEQ ID No:1.

4. A stem cell according to claim 3, wherein said gene consists of a nucleotide sequence with at least 90% identity to the nucleotide sequence set forth in SEQ ID No:1.

5. A stem cell according to claim 4, wherein said gene consists of a nucleotide sequence with at least 95% identity to the nucleotide sequence set forth in SEQ ID No:1.

6. A library of non human stem cells with a modification incorporated into both strands of the allele thereof, wherein the stem cells included in the library have incorporated the modification over the entire genome thereof and wherein the Bloom's syndrome (Blm) gene has been deleted or modified such that the gene does not function and the cells show increased loss of heterozygosity, wherein the Bloom's syndrome (Blm) gene comprises the sequence set forth in SEQ ID NO: 1 or a variant thereof with at least 70% identity to SEQ ID No:1.

7. A library according to claim 6, wherein the stem cell is a non-human embryonic stem cell.

8. A method for producing a non-human stem cell with a modification incorporated into both strands of the alleles thereof, the method comprising the steps of:
A) providing a stem cell;
B) making the Bloom's syndrome gene in the stem cell unfunctional such that the cell shows increased loss of heterozygosity; and
C) inducing mutation in the stem cell
wherein the Bloom's syndrome (Blm) gene comprises the sequence set forth in SEQ ID NO:1 or a variant thereof with at least 70% identity to SEQ ID No:1.

9. A method according to claim 8, wherein the Bloom's syndrome gene is processed so as to be transiently dysfunctional.

10. A method according to claim 9, wherein the Bloom's syndrome gene is processed so as to be transiently dysfunctional in the presence of an agent.

11. A method according to claim 10, wherein the agent is selected from the group consisting of tetracycline, doxycyclin, estrogen derivatives and progesteron derivatives.

12. A method according to claim 8, wherein the induction of mutation is selected from the group consisting of exposure to a mutagen, use of a transposon gene, exposure to ultraviolet and exposure to radioactive rays.

13. A method according to claim 8, further comprising the step of inducing homologous recombination.

14. A method according to claim 13 further comprising the step of inducing homologous recombination in the 4N phase of the cell, thereby inducing cell division after the induction.

15. A method according to claim 8, wherein the stem cell is a non-human embryonic stem cell.

16. A method according to claim 8, wherein the embryonic stem cell is a non-human mammalian embryonic stem cell.

## Patentansprüche

1. Nicht-humane Stammzelle mit einer Modifizierung, eingebracht in beide Stränge der Allele davon, und wobei das Bloom-Syndrom (Blm)-Gen deletiert oder modifiziert wurde, so dass das Gen seine Funktion nicht ausübt und die Zelle einen erhöhten Verlust von Heterozygosität zeigt, wobei das Bloom-Syndrom (Blm)-Gen die Sequenz, welche in SEQ ID Nr.: 1 dargelegt ist, oder eine Variante davon mit mindestens 70 % Identität zu SEQ ID Nr.: 1 umfasst.

2. Nicht-humane Stammzelle gemäß Anspruch 1, welche eine embryonale Stammzelle ist.

3. Stammzelle gemäß Anspruch 1, wobei das Gen aus einer Nukleotidsequenz mit mindestens 80 % Identität zu der in SEQ ID Nr.: 1 dargelegten Nukleotidsequenz besteht.

4. Stammzelle gemäß Anspruch 3, wobei das Gen aus einer Nukleotidsequenz mit mindestens 90 % Identität zu der in SEQ ID Nr.: 1. dargelegten Nukleotidsequenz besteht.

5. Stammzelle gemäß Anspruch 4, wobei das Gen aus einer Nukleotidsequenz mit mindestens 95 % Identität zu der in SEQ ID Nr.: 1 dargelegten Nukleotidsequenz besteht.

6. Bibliothek von nicht-humanen Stammzellen mit einer Modifizierung, eingebracht in beide Stränge des Allels davon, wobei die Stammzellen, welche in der Bibliothek eingeschlossen sind, die Modifizierung über das gesamte Genom davon eingebracht aufweisen und wobei das Bloom-Syndrom (Blm)-Gen deletiert oder modifiziert wurde, so dass das Gen seine Funktion nicht ausübt und die Zellen einen erhöhten Verlust von Heterozygosität zeigen, wobei das Bloom-Syndrom (Blm)-Gen die Sequenz, welche in SEQ ID Nr.: 1 dargelegt ist, oder eine Variante davon mit mindestens 70 % Identität zu SEQ ID Nr.: 1 umfasst.

7. Bibliothek gemäß Anspruch 6, wobei die Stammzelle eine nicht-humane embryonale Stammzelle ist.

8. Verfahren zur Herstellung einer nicht-humanen Stammzelle mit einer Modifizierung, eingebracht in beide Stränge der Allele davon, wobei das Verfahren die folgenden Schritte umfasst:
A) das Bereitstellen einer Stammzelle;
B) das Überführen des Bloom-Syndrom-Gens in der Stammzelle in einen Zustand, so dass es seine Funktion nicht ausübt, so dass die Zelle einen erhöhten Verlust von Heterozygosität zeigt; und
C) das Induzieren einer Mutation in der Stammzelle,
wobei das Bloom-Syndrom (Blm)-Gen die Sequenz, welche in SEQ ID Nr.: 1 dargelegt ist, oder eine Variante davon mit mindestens 70 % Identität zu SEQ ID Nr.: 1 umfasst.

9. Verfahren gemäß Anspruch 8, wobei das Bloom-Syndrom-Gen bearbeitet wird, so dass es transient dysfunktionell ist.

10. Verfahren gemäß Anspruch 9, wobei das Bloom-Syndrom-Gen bearbeitet wird, so dass es in der Gegenwart einer Mittels transient dysfunktionell ist.

11. Verfahren gemäß Anspruch 10, wobei das Mittel aus der Gruppe ausgewählt ist, welche aus Tetracyclin, Doxycyclin, Östrogenderivaten und Progesteronderivaten besteht.

12. Verfahren gemäß Anspruch 8, wobei das Induzieren einer Mutation aus der Gruppe ausgewählt ist, welche aus dem Einwirken eines Mutagens, der Verwendung eines Transposongens, dem Einwirken von ultravioletter Strahlung und der Einwirkung von radioaktiven Strahlen besteht.

13. Verfahren gemäß Anspruch 8, ferner umfassend den Schritt des Induzierens einer homologen Rekombination.

14. Verfahren gemäß Anspruch 13, ferner umfassend den Schritt des Induzierens einer homologen Rekombination in der 4N-Phase der Zelle, damit nach der Induzierung Zellteilung induziert wird.

15. Verfahren gemäß Anspruch 8, wobei die Stammzelle eine nicht-humane embryonale Stammzelle ist.

16. Verfahren gemäß Anspruch 8, wobei die embryonale Stammzelle eine nicht-humane embryonale Säugerstammzelle ist.

## Revendications

1. Cellule souche non-humaine avec une modification incorporée dans les deux brins des allèles de celle-ci et dans laquelle le gène du syndrome de Bloom (Blm) a été délété ou modifié de telle sorte que le gène ne fonctionne pas et la cellule montre une perte accrue d'hétérozygotie, dans laquelle le gène du syndrome de Bloom (Blm) comprend la séquence présentée dans SEQ ID No: 1 ou un variant de celle-ci avec au moins 70% d'identité avec SEQ ID NO: 1.

2. Cellule souche non-humaine selon la revendication 1, qui est une cellule souche embryonnaire.

3. Cellule souche selon la revendication 1, dans laquelle ledit gène est constitué d'une séquence nucléotidique avec au moins 80% d'identité avec la séquence nucléotidique présentée dans SEQ ID No: 1.

4. Cellule souche selon la revendication 3, dans laquelle ledit gène est constitué d'une séquence nucléotidique avec au moins 90% d'identité avec la séquence nucléotidique présentée dans SEQ ID No: 1.

5. Cellule souche selon la revendication 4, dans laquelle ledit gène est constitué d'une séquence nucléotidique avec au moins 95% d'identité avec la séquence nucléotidique présentée dans SEQ ID No: 1.

6. Banque de cellules souches non humaines avec une modification incorporée dans les deux brins de l'allèle de celle-ci, dans laquelle les cellules souches incluses dans la banque ont incorporé la modification sur le génome entier de celle-ci et dans laquelle le gène du syndrome de Bloom (Blm) a été délété ou modifié de telle sorte que le gène ne fonctionne pas et les cellules montrent une perte accrue d'hétérozygotie, dans laquelle le gène du syndrome de Bloom (Blm) comprend la séquence présentée dans SEQ ID No: 1 ou un variant de celle-ci avec au moins 70% d'identité avec SEQ ID No: 1.

7. Banque selon la revendication 6, dans laquelle la cellule souche est une cellule souche embryonnaire non-humaine.

8. Procédé pour produire une cellule souche non-humaine avec une modification incorporée dans les deux brins des allèles de celle-ci, le procédé comprenant les étapes de
A) fournir une cellule souche ;
B) rendre non fonctionnel le gène du syndrome de Bloom dans la cellule souche de telle sorte que la cellule montre une perte accrue d'hétérozygotie ; et
C) induire une mutation dans la cellule souche
dans laquelle le gène du syndrome de Bloom (Blm) comprend la séquence présentée dans SEQ ID No: 1 ou un variant de celle-ci avec au moins 70% d'identité avec SEQ ID No: 1.

9. Procédé selon la revendication 8, dans lequel le gène du syndrome de Bloom est traité de manière à être transitoirement dysfonctionnel.

10. Procédé selon la revendication 9, dans lequel le gène du syndrome de Bloom est traité de manière à être transitoirement dysfonctionnel en présence d'un agent.

11. Procédé selon la revendication 10, dans lequel l'agent est choisi dans le groupe constitué par la tétracycline, la doxycycline, les dérivés des estrogènes et les dérivés de la progestérone.

12. Procédé selon la revendication 8, dans lequel l'induction d'une mutation est choisie dans le groupe constitué par l'exposition à un agent mutagène, l'utilisation d'un gène transposon, l'exposition à des rayons ultraviolets et l'exposition à des rayons radioactifs.

13. Procédé selon la revendication 8, comprenant en outre l'étape d'induction d'une recombinaison homologue.

14. Procédé selon la revendication 13 comprenant en outre l'étape d'induction d'une recombinaison homologue dans la phase 4N de la cellule, induisant par ce moyen une division cellulaire après l'induction.

15. Procédé selon la revendication 8, dans lequel la cellule souche est une cellule souche embryonnaire non-humaine.

16. Procédé selon la revendication 8, dans lequel la cellule souche embryonnaire est une cellule souche embryonnaire de mammifère non-humain.
